# EUROPÄISCHE PATENTSCHRIFT

(11) **EP 0 946 525 B1**
(45) Veröffentlichungstag und Bekanntmachung des Hinweises auf die Patenterteilung: **07.04.2004**
(21) Anmeldenummer: 97951981.6
(22) Anmeldetag: 26.11.1997
(51) Int. Cl.: C07D 257/02

(54) **VERFAHREN ZUR HERSTELLUNG VON METALLKOMPLEXCARBONSÄUREAMIDEN**
METHOD FOR PRODUCING METAL COMPLEX CARBOXYLIC ACID AMIDES
PROCEDE DE PREPARATION D'AMIDES D'ACIDE CARBOXYLIQUE A COMPLEXES METALLIFERES

(30) Priorität: 04.12.1996 DE 19652386
(43) Veröffentlichungstag der Anmeldung: 06.10.1999
(73) Patentinhaber: SCHERING AKTIENGESELLSCHAFT, 13342 Berlin (DE)
(72) Erfinder: SCHMITT-WILLICH, Heribert, D-12161 Berlin (DE); PLATZEK, Johannes, D-12621 Berlin (DE); GRASKE, Klaus-Dieter, D-12169 Berlin (DE); RADÜCHEL, Bernd, D-13465 Berlin (DE)
(86) Internationale Anmeldenummer: PCT/EP1997/006594
(87) Internationale Veröffentlichungsnummer: WO 1998/024775

(56) Entgegenhaltungen:
- EP-A- 0 255 471
- WO-A-93/12097
- WO-A-93/24469

## Beschreibung

Die Erfindung betrifft den in den Ansprüchen gekennzeichneten Gegenstand, das heißt ein neues Verfahren zur Herstellung von Metallkomplexcarbonsäureamiden.

Metallkomplexcarbonsäureamide finden in der medizinischen Diagnostik und Therapie vielfältige Anwendung. So werden z.B. in den Europäischen Patentanmeldungen Nr. 0255 471, 0331616, 0430863, 0481526, 0130934 und in den Internationalen Patentanmeldungen WO 96/02669 und WO 94/28940 derartige Verbindungen und ihre Anwendung als Kontrastmittel, vor allem in der Kernspintomographie (MRI) beschrieben. Ihre Herstellung erfolgt durch Kupplung eines Amins mit der aktivierten Carbonsäure eines Komplexbildners, dessen zusätzlichen Carbonsäuren in der Regel in geschützter Form vorliegen müssen.
(WO-A-9312097 und WO-A-9324469 sind wenig relevant, da sie keine Amidsynthesen offenbaren.)
Häufig werden Anhydride und N-Hydroxysuccinimide verwendet. Nach Abspaltung der vorhandenen Säureschutzgruppen wird das gewünschte Metall eingeführt.

Als Nachteile dieser Methode sind vor allem anzuführen:
a) Unvollständige Kupplung und damit verbundene Trennprobleme der Reaktionsprodukte. Die nicht umgesetzen Amine haben in der Regel unerwünschte pharmakologische Eigenschaften.
b) Unvollständiger Metalleinbau (Komplexierung) in die Komplexbildner-Konjugate. Daraus resultiert eine unerwünschte Antidot-Wirkung (Beeinträchtigung des Herzkreislauf-Systems) sowie eine geringere Wirksamkeit des gebildeten Produkts als Diagnostikum (geringerer Metallgehalt des Kontrastmittels).
c) Eine Schutzgruppenchemie führt immer zu zusätzlichen Reaktionsschritten, in denen die Schutzgruppen wieder entfernt werden müssen. Hierbei kann es sehr leicht zur Beeinträchtigung der pharmakologischen Eigenschaften des gewünschten Produkts kommen. Außerdem fallen bei der Abspaltung der Schutzgruppen große Mengen an Nebenprodukten an, die anschließend entsorgt werden müssen. Daher ist eine Vermeidung von Schutzgruppen gerade für Verfahren, die im Betriebsmaßstab genutzt werden sollen, wünschenswert.
d) Die Ausbeute der nach der Methode des Standes der Technik erhaltenen Metallkomplexcarbonsäureamide ist oft unbefriedigend.
e) Der Einbau des Metalls, vor allem wenn makrocyclische Komplexbildner verwendet werden, muß bei höheren Temperaturen durchgeführt werden. Auch hierdurch erfolgt eine Beeinträchtigung der Reinheit des Reaktionsprodukts.

Der Erfindung liegt somit die Aufgabe zugrunde, ein Verfahren zur Herstellung von Metallkomplexcarbonsäureamiden, das die oben genannten Nachteile vermeidet oder zumindest vermindert, zur Verfügung zu stellen.

Es wurde gefunden, daß überraschenderweise diese Aufgabe durch die vorliegende Erfindung, d.h. durch das Verfahren zur Herstellung von Metallkomplexcarbonsäureamiden, dadurch gekennzeichnet, daß ein Metallkomplexcarbonsäure-Gemisch bestehend aus der Metallkomplexcarbonsäure und mindestens einem lösungsvermittelnden Stoff in Dimethylsulfoxid (DMSO) mit einem wasserabspaltenden Reagenz gegebenenfalls unter Zusatz eines Kupplungs-Hilfsstoffs vorbehandelt wird und anschließend mit einem Amin der allgemeinen Formel I

A(NH₂)ₙ (I),

worin
A für den Rest eines natürlichen oder synthetischen Amins und
n für die Zahlen 1 bis 100 stehen,
umgesetzt wird, gelöst wird.

Das in die Kupplungsreaktion eingesetzte Gemisch aus Metallkomplexcarbonsäure und mindestens einem Iösungsvermittelndem Stoff in einer Menge bis zu 5, vorzugsweise 0,5-2 Moläquivalenten bezogen auf die Metallkomplexcarbonsäure kann sowohl in einer vorgeschalteten Reaktionsstufe hergestellt und (z.B. durch Eindampfen, Gefriertrocknung oder Sprühtrocknung einer wäßrigen oder mit Wasser mischbaren Lösung der Bestandteile oder durch Fällung mit einem organischen Lösungsmittel aus einer derartigen Lösung) isoliert werden und anschließend in DMSO mit wasserabspaltenden Reagenz und gegegebenenfalls einem Kupplungs-Hilfsstoff umgesetzt werden als auch in situ durch Zusatz von lösungsvermittelndem/n Stoff(en) zur DMSO-Suspension von Metallkomplexcarbonsäure, wasserabspaltendem Reagenz und gegebenenfalls einem Kupplungs-Hilfsstoff gebildet werden.

Die nach einem dieser Verfahren hergestellte Reaktionslösung wird zur Vorbehandlung (Säureaktivierung) 1 bis 24, vorzugsweise 3 bis 12 Stunden bei Temperaturen von 0 bis 50° C, vorzugsweise bei Raumtemperatur, gehalten. Anschließend wird ein Amin der allgemeinen Formel I ohne Lösungsmittel oder gelöst, vorzugsweise in DMSO, in Wasser oder in mit Wasser gemischten Lösungsmitteln zugesetzt. Zur Amidkupplung wird die so erhaltene Reaktionslösung bei Temperaturen von 0 bis 70° C, vorzugsweise 30 bis 60° C, 1 bis 48, vorzugsweise 8 bis 24 Stunden gehalten.

In einigen Fällen hat es sich als vorteilhaft erwiesen, das Amin in Form seiner Salze, z.B. als Hydrobromid oder Hydrochlorid in die Reaktion einzusetzen. Zur Freisetzung des Amins wird eine Base wie z.B. Triethylamin, Diisopropylethylamin, N-Methylmorpholin, Pyridin, Tripropylamin, Tributylamin, Lithiumhydroxid, Lithiumcarbonat, Natriumhydroxid oder Natriumcarbonat zugesetzt.

Die Isolierung des Reaktionsprodukts erfolgt nach den dem Fachmann bekannten Methoden, vorzugsweise durch Ausfällung mit organischen Lösungsmitteln, vorzuasweise Aceton, 2-Butanon, Diethylether, Essigester, Methyl-t.-butylether, Isopropanol oder deren Mischungen. Die weitere Reinigung kann beispielsweise durch Chromatographie, Kristallisation oder Ultrafiltration erfolgen.

Als lösungsvermittelnde Stoffe sind Alkali-, Erdalkali-, Trialkylammonium-, Tetraalkylammoniumsalze, Harnstoffe, N-Hydroxyimide, Hydroxyaryltriazole, substituierte Phenole und Salze von heterocyclischen Aminen geeignet, Beispielhaft genannt seien: Lithiumchlorid, Lithiumbromid, Lithiumjodid, Natriumbromid, Natriumjodid, Lithiummethansulfonat, Natriummethansulfonat, Lithium-p-toluolsulfonat, Natrium-ptoluolsulfonat, Kaliumbromid, Kaliumjodid, Natriumchlorid, Magnesiumbromid, Magnesiumchlorid, Magnesiumjodid, Tetraethylammonium-p-toluolsulfonat, Tetramethylammonium-p-toluolsulfonat, Pyridinium-p-toluolsulfonat, Triethylammonium-ptoluolsulfonat, 2-Morpholinoethylsulfonsäure, 4-Nitrophenol, 3,5-Dinitrophenol, 2,4-Dichlorphenol, N-Hydroxysuccinimid, N-Hydroxyphthalimid, Harnstoff, Tetramethylharnstoff, N-Methylpyrrolidon, Formamid sowie cyclische Harnstoffe, wobei die fünf erstgenannten bevorzugt sind.

Als wasserabspaltende Reagenzien dienen alle dem Fachmann bekannten Mittel (siehe z.B. Houben-Weyl, Methode der organischen Chemie, Bd. XV/2, Georg Thieme-Verlag, Stuttgart, 1974 und J.Chem. Research (S) 1996, 302).

Beispielhaft genannt seien Carbodiimide und Onium-Reagenzien wie z.B. Dicyclohexylcarbodiimid (DCCI), 1-Ethyl-3-(3-dimethylaminopropyl)-carbodiimidhydroxychlorid (EDC), Benzotriazol-1-yloxytris(dimethylamino)-phosphoniumhexafluorophosphat (BOP) und O-(benzotriazol-1-yl)-1,1,3,3-tetramethyluroniumhexafluorophosphat (HBTU), vorzugsweise DCCI.

Als gegebenenfalls zu verwendende Kupplungs-Hilfsstoffe sind alle dem Fachmann bekannten geeignet (Houben-Weyl, Methoden der organischen Chemie, Bd. XV/2, Georg Thieme-Verlag, Stuttgart, 1974). Beispielhaft genannt seien 4-Nitrophenol, N-Hydroxysuccinimid, 1-Hydroxybenzotriazol, 1-Hydroxy-7-azabenzotriazol, 3,5-Dinitrophenol und Pentafluorphenol, Bevorzugt sind 4-Nitrophenol und N-Hydroxysuccinimid, besonders bevorzugt ist dabei das erstgenannte Reagenz.

Als Amine der allgemeinen Formel I sind Monoamine (n = 1) und Polyamine (n=2-100) geeignet. Von den Polyaminen sind diejenigen mit n = 2 bis 50 bevorzugt, besonders bevorzugt diejenigen mit n = 12 bis 36. Beispielhaft genannt seien Dendrimere (wie z.B. in Polymer Journal 17, 117 (1985), US-4,587,329, EP 0430863, WO 96/01655 genannt) Proteine, Polylysine (wie z.B. in EP 0481526 und EP 0331616 genannt), Aminopolysaccharide (wie z.B. in WO 94/28940 genannt), Polyvinylamine, Polyalkylamine, Poly[N(2-Aminoethyl)]methacrylamide, Polynucleotide (s. z.B. WO 96/02669), Antisense-Polynucleotide, Polypeptide, Antibiotika, Nukleoside, Aminoterpene, Aminoporphyrine (s. z.B. WO 94/07894), Aminosteroide und Aminozucker, Bevorzugt sind Dendrimere, vor allem solche, die im Rest A der allgemeinen Formel I keine protonierbaren Aminogruppen enthalten.Besonders bevorzugt sind die in der WO 96/01655 genannten Kaskaden-Polymeramine, ganz besonders bevorzugt ist das 24mer Polyamin auf der Basis des N,N,N',N',N",N"-Hexakis[2-(trilysyl-amino)-ethyl]-trimesinsäuretriamids (s. Beispiel 12).

Als Metallkomplexcarbonsäuren sind lineare (z.B. die in DE 19507822 und EP 0450742 genannten) und makrocyclische (z.B. die in EP 0485045 genannten) Verbindungen geeignet. Bevorzugt sind Makrocyclen auf der Basis des 1,4,7,10-Tetraazacyclododekan-Gerüstes. Besonders bevorzugt sind solche der allgemeinen Formel II 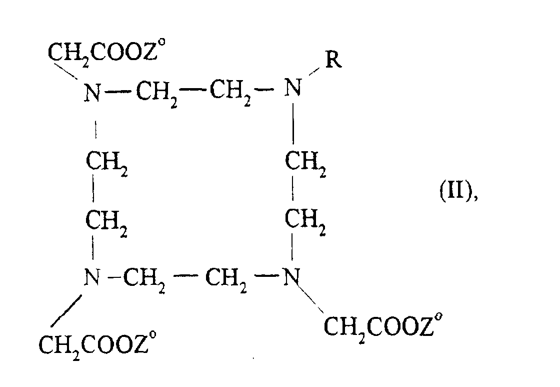 wobei
- Z^{O}: für ein Metallionenäquivalent der Ordnungszahlen 25, 26, 39, 57-71, 83 und
- R: für eine CHX¹-CO-NH-CHY¹-(CH₂)_{f}-COOH-Gruppe steht, worin X¹ und Y¹ unabhängig voneinander ein Wasserstoffatom, einen geradkettigen oder verzweigten C₁-C₇-Alkylrest, eine Phenyl- oder Benzylgruppe und
f die Ziffern 0 bis 9 bedeuten.

Für die Reste X¹ bzw. Y¹ seien beispielhaft genannt: Methyl, Ethyl, Propyl, Butyl bzw. Wasserstoff, Methyl, Isopropyl, Phenyl und Benzyl. Bevorzugt sind Methyl bzw. Wasserstoff.

Der Index f steht bevorzugt für die Ziffern 0, 1 oder 2.

Von den oben genannten Lanthaniden sind Gadolinium und Dysprosium bevorzugt.
Als Komplexbildner der allgemeinen Formel II (Z^{O} = Wasserstoff) ist die 10-[4-Carboxy-1-methyl-2-oxo-3-azabutyl]-1,4,7,10-tetraazacyclododekan-1,4,7-triessigsäure besonders bevorzugt.

Die Synthese dieser Verbindungen erfolgt dadurch, daß man Verbindungen der allgemeinen Formel III 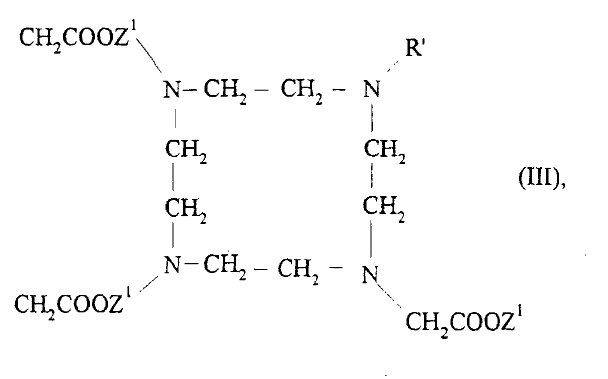 worin
- R': die Bedeutung von R hat, wobei die darin enthaltene Carboxylgruppe gegebenenfalls in geschützter Form vorliegt und
- Z¹: für ein Wasserstoffatom oder eine Carboxylschutzgruppe steht, nach Abspaltung der gegebenenfalls vorhandenen Carboxylschutzgruppen, in an sich bekannter Weise mit einem Metalloxid oder Metallsalz eines Elements der Ordnungszahlen 25, 26, 39, 57-71, 83 umsetzt.

Die Einführung der gewünschten Metallionen erfolgt in der Weise, wie sie z.B. in den Patentschriften EP 71564, EP 130934 und DE-3401052 offenbart worden ist, indem man das Metalloxid oder ein Metallsalz (beispielsweise das Nitrat, Acetat, Carbonat, Chlorid oder Sulfat) des Elements der gewünschten Ordnungszahlen in Wasser und/oder einem niederen Alkohol (wie Methanol, Ethanol oder Isopropanol) löst oder suspendiert und mit einer Lösung oder Suspension der äquivalenten Menge des Komplexbildners der allgemeinen Formel III umsetzt.

Falls Z¹ für eine Säureschutzgruppe steht, kommen z.B. geradkettige oder verzweigte C₁-C₆-Alkyl-, Aryl- und Aralkylgruppen, beispielsweise die Methyl-, Ethyl-, Propyl-, Butyl-, Phenyl-, Benzyl-, Diphenylmethyl-, Triphenylmethyl-, bis(p-Nitrophenyl)-methylgruppe sowie Trialkylsilylgruppen in Frage. Bevorzugt ist die t-Butylgruppe.

Die Abspaltung der Schutzgruppen erfolgt nach den dem Fachmann bekannten Verfahren, beispielsweise durch Hydrolyse, Hydrogenolyse, alkalische Verseifung der Ester mit Alkali in wässrig-alkoholischer Lösung bei Temperaturen von 0° bis 50° C, saure Verseifung mit Mineralsäuren oder im Fall von z.B. tert.-Butylestern mit Hilfe von Trifluoressigsäure.[Protective Groups in Organic Synthesis, 2nd Edition, T.W. Greene and P.G.M. Wuts, John Wiley and Sons, Inc. New York, 1991].

Verbindungen der allgemeinen Formel III kann man erhalten durch Umsetzen von α-Halogencarbonsäureestern oder -säuren der allgemeinen Formel IV

Hal-CH₂-CO₂Z¹ (IV),

worin
Z¹ die obengenannte Bedeutung hat und Hal für Chlor-, Brom oder Iod steht,
mit Verbindungen der allgemeinen Formel V 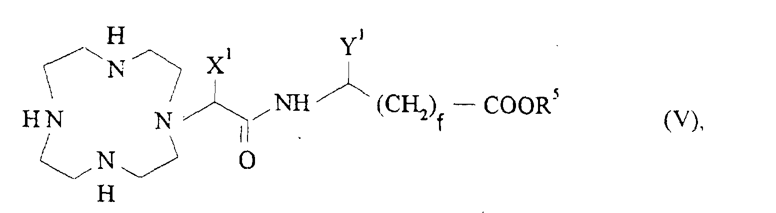 worin
R⁵ für ein Wasserstoffatom, oder eine Säureschutzgruppe steht und
X¹, Y¹ und f die oben genannte Bedeutung haben.

Stehen Z¹ und R⁵ jeweils für eine Säureschutzgruppe so können diese unterschiedliche Bedeutung haben, so daß Z¹ (z.B. Benzyl) gegebenenfalls selektiv (z.B. durch Hydrogenolyse) in Gegenwart von R⁵-Schutzgruppen (z.B. t-Butyl) abgespalten werden kann.

Steht Z¹ für eine Säureschutzgruppe, so erfolgt die Umsetzung bevorzugt in Lösungsmitteln wie Methylenchlorid, Dimethylformamid, Acetonitril, Tetrahydrofuran, Dioxan, Chloroform, niederen Alkoholen wie Methanol, Ethanol und Isopropanol, sowie Mischungen aus den oben genannten Lösungsmitteln mit Wasser.

Bei Verwendung einer Halogencarbonsäure als Edukt wird bevorzugt in Wasser gearbeitet.

Als Säurefanger dienen organische Basen wie Pyridin, Triethylamin oder Diisopropylethylamin oder anorganische Basen wie Natriumhydroxid, Lithiumhydroxid, Kaliumhydroxid, Calciumhydroxid oder Natriumcarbonat, Kaliumcarbonat, Natriumhydrogencarbonat oder Lithiumcarbonat. Die Alkylierung wird bei Temperaturen zwischen 0-100° C durchgeführt, bevorzugt jedoch bei 20-80° C.

Verbindungen der allgemeinen Formel V werden erhalten durch Umsetzung von Cyclen (Formel VI) 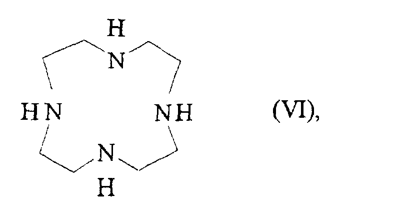 mit Verbindungen der allgemeinen Formel VII 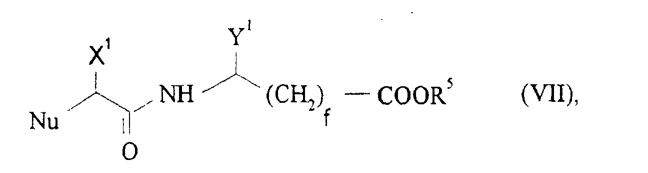 worin
X¹, Y¹, R⁵ und f die oben genannte Bedeutung haben und Nu für ein Nucleofug steht. Als Nucleofuge seien Chlorid, Bromid, Jodid, Mesylat, Tosylat oder Triflat genannt.

Die Umsetzung erfolgt in Lösungsmitteln wie Chloroform, Methylenchlorid, Tetrahydrofuran, Dioxan, Dimethylformamid, Dimethylsulfoxid oder auch in Wasser bei Temperaturen von 0° C bis 100° C, bevorzugt jedoch bei 20° - 60° C. Es kann, falls erwünscht, eine organische oder anorganische Base zugesetzt werden. Beispielhaft genannt seien Triethylamin, Pyridin, Natriumcarbonat, Natriumhydroxid oder Kaliumhydroxid.

Verbindungen der allgemeinen Formel VII erhält man durch Umsetzen von Verbindungen der allgemeinen Formel VIII 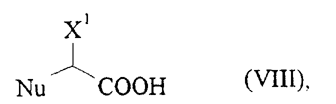 worin
Nu und X¹ die oben genannte Bedeutung haben, mit Verbindungen der allgemeinen Formel IX 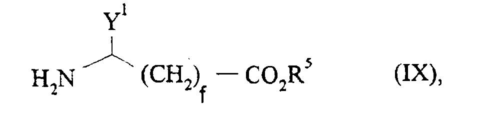 worin
Y¹, fund R⁵ die oben angegebene Bedeutung haben.

Die Umsetzung erfolgt nach den dem Fachmann bekannten Methoden aus der Peptidchemie. So kann beispielsweise aus der Säure der allgemeinen Formel VIII ein Derivat wie z.B. ein Säurechlorid, Säurebromid oder Aktivester (wie z.B. NHS-Ester) hergestellt weden, das mit einer Aminosäure (gegebenenfalls terminalgeschützt) kondensiert wird.

Verbindungen der allgemeinen Formel VIII, sowie deren Säurechloride und Säurebromide sind käuflich erhältlich. Verbindungen der allgemeinen Formel IX sind ebenfalls als freie Aminosäuren oder in geschützter Form käuflich erhältlich.

Alternativ können Verbindungen der allgemeinen Formel III durch Umsetzung von Verbindungen der allgmeinen Formel X 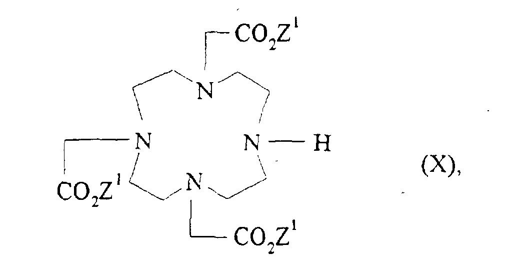 worin Z¹ die oben genannte Bedeutung besitzt (s. z.B. EP 0255471), mit Verbindungen der allgemeinen Formel VII, nach Abspaltung der gegebenenfalls vorhandenen Säureschutzgruppen, erhalten werden.

Die Umsetzung erfolgt in Lösungsmitteln wie beispielsweise Acetonitril, Dimethylformamid, Tetrahydrofuran, Dioxan oder niederen Alkoholen wie Methanol, Ethanol oder i-Propanol sowie Mischungen dieser mit Wasser; es kann aber auch in reinem Wasser gearbeitet werden. Man arbeitet im allgemeinen bei Temperaturen von 20° C - 100° C.

Als Säurefänger werden organische oder anorganische Basen verwendet. Beispielhaft genannt seien Triethylamin, Pyridin, 4-Dimethylaminopyridin, Natriumhydroxid, Kaliumhydroxid, Kaliumcarbonat, Natriumcarbonat. Es können auch Metallhydride wie Natriumhydrid, Calciumhydrid eingesetzt werden, jedoch nur bei aprotischen Lösungsmitteln.

Der Zusatz katalytischer Mengen eines Iodids hat sich als vorteilhaft erwiesen. Beispielhaft genannt seien Natriumiodid, Kaliumiodid, Lithiumiodid oder Tetrabutylammoniumiodid.

Die Reinigung der Metallkomplexe der allgemeinen Formel II erfolgt beispielsweise durch Chromatographie an Kieselgel oder RP-18.

Die meisten der Metallkomplexe der allgemeinen Formel II können aus Alkoholen wie Methanol, Ethanol oder Isopropanol kristallisiert werden oder auch aus deren Mischungen mit Wasser.

Es hat sich auch als günstig erwiesen die Metallkomplexe in Alkoholen oder Mischungen von Alkoholen mit Wasser zu lösen und durch Zutropfen von Aceton auszufällen.

Das Trocknen der Metallkomplexcarbonsäuren geschieht vorteilhafter Weise im Vakuum bei Temperaturen von 20° - 200° C, bevorzugt 50° - 130° C, innerhalb von ca. 6 Stunden bis zu 3 Tagen.

Die so erhaltenen Metallkomplexcarbonsäuren der allgemeinen Formel II werden unter Feuchtigkeitsausschluß gelagert und können direkt in eine Kupplungsreaktion eingesetzt werden.

Das erfindungsgemäße Verfahren zeichnet sich aus durch praktisch vollständige Kupplung der Amine mit den Metallkomplexcarbonsäuren, so daß keine Probleme hinsichtlich der Abtrennung unumgesetzter bzw. partiell umgesetzter Edukte auftreten. Auch enthalten die so gebildeten Amid-Konjugate praktisch keine metallfreien Komplexbildner-Einheiten. Beides führt zu physiologisch besser verträglichen Verbindungen als die nach dem Stand der Technik herstellbaren Konjugate.

Da in dem erfindungsgemäßen Verfahren keine Schutzgruppen eingeführt bzw. abgespalten werden müssen und der das Molekül belastende (hohe Temperatur, niedriger pH-Wert) Einbau des Metalls in der letzten Reaktionsstufe vermieden wird, werden die Produkte in hoher Reinheit erhalten. Hinzu kommt, daß die Ausbeute des erfindungsgemäßen Verfahrens überraschend hoch ist.

Ein weiterer Vorteil des erfindungsgemäßen Verfahrens liegt darin, daß das Verfahren auch für Umsetzungen im Betriebsmaßstab geeignet ist, da die Umsetzung in hochkonzentrierter Lösung durchgeführt werden kann.

Die nachfolgenden Beispiele 1 bis 11 (Zwischenprodukte) dienen zur Erläuterung der Synthese von Metallkomplexcarbonsäuren der allgemeinen Formel II:

### Beispiel 1

### a) N-(2-Brompropionyl)glycin-benzylester

Zu 100 g (296,4 mmol) Glycinbenzylester-p-Toluolsulfonsäuresalz und 33,0 g (326,1 mmol) Triethylamin in 400 ml Methylenchlorid tropft man bei 0 °C 55,9 g (326,1 mmol) 2-Brompropionsäurechlorid zu. Man läßt die Temperatur nicht über 5 °C kommen. Nach beendeter Zugabe wird eine Stunde bei 0 °C gerührt, anschließend 2 Stunden bei Raumtemperatur. Man setzt 500 ml Eiswasser zu und stellt die Wasserphase mit 10 % aqu. Salzsäure auf pH 2. Die organische Phase wird abgetrennt, je einmal mit 300 ml 5 % aqu. Sodalösung und 400 ml Wasser gewaschen. Man trocknet die organische Phase über Magnesiumsulfat und dampft im Vakuum zur Trockne ein. Der Rückstand wird aus Diisopropylether umkristallisiert.
Ausbeute: 68,51 g (75 % d. Th.) eines farblosen kristallinen Pulvers
Schmelzpunkt: 69-70°C

| Elementaranalyse: | | | | |
|---|---|---|---|---|
| ber. | C 48,02 | H 4,70 | N 4,67 | Br 26,62 |
| gef. | C 47,91 | H 4,82 | N 4,51 | Br 26,47 |

### b) 1-[4-(Benzyloxycarbonyl)-1-methyl-2-oxo-3-azabutyl]-1,4,7,10-tetraazacyclododecan

Zu 55,8 g (324,4 mmol) 1,4,7,10-Tetraazacyclododecan, gelöst in 600 ml Chloroform, gibt. man 50 g (162,2 mmol) der Titelverbindung aus Beispiel 1a) und rührt über Nacht bei Raumtemperatur. Man gibt 500 ml Wasser zu, trennt die organische Phase ab und wäscht sie noch jeweils 2 mal mit 400 ml Wasser. Man trocknet die organische Phase über Magnesiumsulfat und dampft im Vakuum zur Trockne ein. Der Rückstand wird an Kieselgel chromatographiert (Laufmittel: Chloroform/Methanol/aqu. 25 % Ammoniak = 10/5/1).
Ausbeute. 40,0 g [63 % d. Th.bezogen auf eingesetztes 1a)] eines leicht gelblichen zähen Öls.

| Elementaranalyse: | | | |
|---|---|---|---|
| ber. | C 61,36 | H 8,50 | N 17,89 |
| gef. | C 61,54 | H 8,68 | N 17,68 |

### c) 10-[4-(Benzyloxycarbonyl)-1-methyl-2-oxo-3-azabutyl]-1,4,7-tris(tert.-butoxycarbonylmethyl)-1,4,7,10-tetraazacyclododecan (Natriumbromid-Komplex)

Zu 20 g (51,08 mmol) der Titelverbindung aus Beispiel 1b) und 17,91 (169 mmol) Natriumcarbonat in 300 ml Acetonitril gibt man 33 g (169 mmol) Bromessigsäure-tert.-butylester zu und rührt 24 Stunden bei 60 °C. Man kühlt auf 0 °C ab, filtriert von den Salzen ab und dampft das Filtrat zur Trockne ein. Der Rückstand wird an Kieselgel chromatographiert (Laufmittel: Essigsäureethylester/Ethanol: 15/1). Die das Produkt enthaltenden Fraktionen werden eingedampft und der Rückstand aus Diisopropylether umkristallisiert.
Ausbeute: 34,62 g (81 % d. Th.) eines farblosen kristallinen Pulvers
Schmelzpunkt: 116 - 117 °C

| Elementaranalyse: | | | | | |
|---|---|---|---|---|---|
| ber. | C 54,54 | H 7,59 | N 8,37 | Na 2,74 | Br 9,56 |
| gef. | C 54,70 | H 7,65 | N 8,24 | Na 2,60 | Br 9,37 |

### d) 10-(4-Carboxy-1-methyl-2-oxo-3-azabutyl)-1,4,7-tris(tert.-butoxycarbonylmethyl)-1,4,7,10-tetraazacyclododecan (Natriumbromid-Komplex)

30 g (35,85 mmol) der Titelverbindung aus Beispiel 1c werden in 500 ml Isopropanol gelöst und 3 g Palladiumkatalysator (10 % Pd/C) hinzugegeben. Man hydriert über Nacht bei Raum-temperatur. Es wird vom Katalysator abfiltriert, das Filtrat im Vakuum zur Trockne eingedampft und aus Aceton umkristallisiert.
Ausbeute: 22,75 g (85 % d. Th.) eines farblosen kristallinen Pulvers
Schmelzpunkt: 225 °C (Zers.)

| Elementaranalyse: | | | | | |
|---|---|---|---|---|---|
| ber. | C 49,86 | H 7,69 | N 9,38 | Na 3,07 | Br 10,71 |
| gef. | C 49,75 | H 7,81 | N 9,25 | Na 2,94 | Br 10,58 |

### e) 10-[4-Carboxy-1-methyl-2-oxo-3-azabutyl]-1,4,7,10-tetraazacyclododecan-1,4,7-triessigsäure

77 g (103,1 mmol) der Titelverbindung aus Beispiel 1d werden in 500 ml Trifluoressigsäure gelöst und 3 Stunden bei Raumtemperatur gerührt. Man dampft zur Trockne ein, nimmt den Rückstand in 300 ml Wasser auf und gibt die Lösung auf eine Säule, gefüllt mit Reillex® 425 PVP. Man eluiert mit Wasser. Die produkthaltigen Fraktionen werden vereinigt und zur Trockne eingedampft, der Rückstand wird aus Methanol/Aceton umkristallisiert.
Ausbeute: 44,04 g (84 % d. Th.) eines farblosen, hygroskopischen Feststoffes
Wassergehalt: 6,5 %

| Elementaranalyse (berechnet auf wasserfreie Substanz): | | | |
|---|---|---|---|
| ber. | C 47,99 | H 6,99 | N 14,73 |
| gef. | C 47,83 | H 7,12 | N 14,55 |

### f) Gadolinium-Komplex der 10-[4-Carboxy-1-methyl-2-oxo-3-azabutyl]-1,4,7,10-tetraazacyclododecan-1,4,7-triessigsäure

Zu 40 g (84,12 mmol) der Titelverbindung aus Beispiel 1e, gelöst in 400 ml Wasser, gibt man 15,27 g (42,06 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 50,53 g (93 % d. Th.) eines farblosen kristallinen Pulvers
Wassergehalt: 2,5 %

| Elementaranalyse (berechnet auf wasserfreie Substanz): | | | | |
|---|---|---|---|---|
| ber. | C 36,24 | H 4,80 | N 11,12 | Gd 24,97 |
| gef. | C 36,35 | H 4,95 | N 10,98 | Gd 24,80 |

### Beispiel 2

### Dysprosiumkomplex der 10-[4-Carboxy-1-methyl-2-oxo-3-azabutyl]-1,4,7,10-tetraazacyclododecan-1,4,7-triessigsäure

Zu 20 g (42,06 mmol) der Titelverbindung aus Beispiel 1e, gelöst in 200 ml Wasser, gibt man 7,84 g (21,03 mmol) Dysprosiumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 24,98 (91 % d. Th.) eines farblosen kristallinen Pulvers
Wassergehalt: 2,7 %

| Elementaranalyse (berechnet auf wasserfreie Substanz): | | | | |
|---|---|---|---|---|
| ber. | C 35,94 | H 4,76 | N 11,03 | Dy 25,59 |
| gef. | C 35,85 | H 4,91 | N 10,90 | Dy 25,42 |

### Beispiel 3

### Ytterbiumkomplex der 10-[4-Carboxy-1-methyl-2-oxo-3-azabutyl]-1,4,7,10-tetraazacyclododecan-1,4,7-triessigsäure

Zu 20 g (42,06 mmol) der Titelverbindung aus Beispiel 1e, gelöst in 200 ml Wasser, gibt man 8,29 g (21,03 mmol) Ytterbiumoxid und erwärmt 3 Tage auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 21,79 (78 % d. Th.) eines farblosen kristallinen Pulvers
Wassergehalt: 2,8 %

| Elementaranalyse (berechnet auf wasserfreie Substanz): | | | | |
|---|---|---|---|---|
| ber. | C 35,35 | H 4,68 | N 10,85 | Yb 26,81 |
| gef. | C 35,25 | H 4,79 | N 10,68 | Yb 26,61 |

### Beispiel 4

### a) N-(2-Brombutyryl)-glycinbenzylester

Zu 100 g (296,4 mmol) Glycinbenzylester p-Toluolsulfonsäuresalz und 89,98 g (889,2 mmol) Triethylamin in 500 ml Methylenchlorid tropft man bei 0°C 65,96 g (355,7 mmol) α-Brom-Buttersäurechlorid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 500 ml 5 %iger aqu. Salzsäure extrahiert, über Magnesiumsulfat getrocknet und im Vakuum zur Trockne eingedampft. Der Rückstand wird aus Di-isopropylether umkristallisiert.
Ausbeute: 75,43 g (81 % d. Th.) eines farblosen kristallinen Pulvers

| Elementaranalyse: | | | | |
|---|---|---|---|---|
| ber. | C 49,70 | H 5,13 | N 4,46 | Br 25,43 |
| gef. | C 49,51 | H 5,27 | N 4,31 | Br 25,28 |

### b) 10-[4-(Benzyloxycarbonyl)-1-ethyl-2-oxo-3-azabutyl]-1,4,7,10-tetraazacyclododecan-1,4,7-triessigsäure-tri-tert-butylester

Zu 50 g (159,14 mmol) der Titelverbindung aus Beispiel 4a, 36,98 g (79,6 mmol) 1,4,7-tris(tert.butoxy-carbonylmethyl)-1,4,7,10-tetraazacyclododecan (= DO3A-tri-tert.butylester), 44 g (318,4 mmol) Kaliumcarbonat und 1 g (60 mmol) Kaliumjodid gibt man 500 ml Acetonitril und erhitzt 12 Stunden unter Rückfluß. Man filtriert von den Salzen ab und dampft das Filtrat im Vakuum zur Trockne ein. Der Rückstand wird in 800 ml Dichlormethan gelöst und 2 mal mit je 300 ml 5 %iger aqu. Natriumcarbonat-Lösung extrahiert. Die organische Phase wird über Magnesiumsulfat getrocknet und eingedampft. Nach Chromatographie an Kieselgel (Laufmittel: Dichlormethan/ Methanol= 20:1) erhält man 19,11 g der Titelverbindung (32,1 % d. Th.) als farblosen Schaum.

| Elementaranalyse: | | | |
|---|---|---|---|
| ber. | C 62,63 | H 8,76 | N 9,36 |
| gef. | C 62,51 | H 8,91 | N 9,18 |

### c) 10-(4-Carboxy-1-ethyl-2-oxo-3-azabutyl)-1,4,7,10-tetraazacyclododecan-1,4,7-triessigsäure-tri-tert-butylester

19 g (25,40 mmol) der Titelverbindung aus Beispiel 4b löst man in 300 ml Isopropanol und gibt 2 g Palladiumkatalysator (10 % Pd/C) zu. Man hydriert über Nacht bei Raum-temperatur.
Es wird vom Katalysator abfiltriert und das Filtrat zur Trockne eingedampft.
Ausbeute: 16,54 g (99 % d. Th.) eines zähflüssigen Öls

| Elementaranalyse: | | | |
|---|---|---|---|
| ber. | C 58,43 | H 9,04 | N 10,65 |
| gef. | C 58,65 | H 9,27 | N 10,47 |

### d) 10-(4-Carboxy-1-ethyl-2-oxo-3-azabutyl)-1,4,7,10-tetraazacyclododecan-1,4,7-triessigsäure

16 g (24,32 mmol) der Titelverbindung aus Beispiel 4c werden in 100 ml Trifluoressigsäure gelöst und 3 Stunden bei Raumtemperatur gerührt. Man dampft zur Trockne ein, nimmt den Rückstand in 50 ml Wasser auf und gibt die Lösung auf eine Säule, gefüllt mit Reillex® 425 PVP. Man eluiert mit Wasser. Die produkthaltigen Fraktionen werden vereinigt und zur Trockne eingedampft, der Rückstand wird aus Methanol/Aceton umkristallisiert.
Ausbeute: 10,10 g (79 % d. Th.) eines farblosen, hygroskopischen Feststoffes
Wassergehalt: 6,9 %

| Elementaranalyse (berechnet auf wasserfreie Substanz): | | | |
|---|---|---|---|
| ber. | C 49,07 | H 7,21 | N 14,31 |
| gef. | C 49,28 | H 7,39 | N 14,15 |

### e) Gadoliniumkomplex der 10-(4-Carboxy-1-ethyl-2-oxo-3-azabutyl)-1,4,7,10-tetraazacyclododecan-1,4,7-triessigsäure

Zu 9 g (18,38 mmol) der Titelverbindung aus Beispiel 4d, gelöst in 70 ml Wasser, gibt man 3,33 g (9,19 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 11,44 g (94 % d. Th.) eines farblosen kristallinen Pulvers
Wassergehalt: 2,8 %

| Elementaranalyse (berechnet auf wasserfreie Substanz): | | | | |
|---|---|---|---|---|
| ber. | C 37,32 | H 5,01 | N 10,88 | Gd 24,43 |
| gef. | C 37,15 | H 5,21 | N 10,65 | Gd 24,25 |

### Beispiel 5

Dysprosiumkomplex der 10-(4-Carboxy-1-ethyl-2-oxo-3-azabutyl)-1,4,7,10-tetraazacyclododecan-1,4,7-triessigsäure

Zu 10 g (20,43 mmol) der Titelverbindung aus Beispiel 4d gelöst in 80 ml Wasser gibt man 3,81 g (10,21 mmol) Dysprosiumoxid und erwärmt 3 h auf 90°C. Man dampft zur Trockne ein (Vakuum und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 12,40 g (91 % d. Th.) eines farblosen, kristallinen Pulvers
Wassergehalt: 2,7 %

| Elementaranalyse (berechnet auf wasserfreie Substanz): | | | | |
|---|---|---|---|---|
| ber. | C 37,01 | H 4,97 | N 10,79 | Dy 25,04 |
| gef. | C 36,85 | H 5,13 | N 10,61 | Dy 24,87 |

### Beispiel 6

### a) N-[2-Brom-2-phenyl-acetyl]-glycinsäure-tert.-butylester

Zu 50 g (296,5 mmol) Glycin-tert.-butylester Hydrochloridsalz und 90 g (889,5 mmol) Triethylamin in 500 ml Methylenchlorid tropft man bei 0°C 72.69 g (311.3 mmol) α-Brom-Phenylessigsäurechlorid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 500 ml 5 %iger aqu. Salzsäure extrahiert, über Magnesiumsulfat getrocknet und im Vakuum zur Trockne eingedampft. Der Rückstand wird aus Di-isopropylether/n-Hexan umkristallisiert.
Ausbeute: 78,8 g (81 % d. Th.)

| Elementaranalyse: | | | | |
|---|---|---|---|---|
| ber. | C 51,23 | H 5,53 | N 4,27 | Br 24,35 |
| gef. | C 51,15 | H 5,66 | N 4,11 | Br 24,18 |

### b) 1-[4-(tert.-butoxycarbonyl)-oxo-1-phenyl-3-azabutyl]-4,7,10-tris(tert.butoxycarbonylmethyl)-1,4,7,10-tetraazacyclododecan

Zu 50 g (159,14 mmol) der Titelverbindung aus Beispiel 6a, 53,12 g (114,3 mmol) 1,4,7-tris(tert.butoxy-carboxymethyl)-1,4,7,10-tetraazacyclododecan (= DO3A-tri-tert.butylester), 63,16 g (457,0 mmol) Kaliumcarbonat und 1 g (6 mmol) Kaliumjodid gibt man 500 ml Acetonitril und erhitzt 12 Stunden unter Rückfluß. Man filtriert von den Salzen ab und dampft das Filtrat im Vakuum zur Trockne ein. Der Rückstand wird in 1000 ml Dichlormethan gelöst und 2 mal mit je 400 ml 5 %iger aqu. Natriumcarbonat-Lösung extrahiert. Die vereinigten organischen Phasen werden über Magnesiumsulfat getrocknet und eingedampft. Nach Chromatographie an Kieselgel (Laufmittel: Dichlormethan/ Methanol= 20: 1) erhält man 27 g der Titelverbindung (31 % d. Th.) als farblosen Schaum.

| Elementaranalyse: | | | |
|---|---|---|---|
| ber. | C 63,05 | H 8,86 | N 9,19 |
| gef. | C 62,91 | H 8,98 | N 9,02 |

### c) 1-(4-carboxy-2-oxo-1-phenyl-3-azabutyl)-4,7,10-tris(carboxymethyl)-1,4,7,10-tetraazacyclododecan

26 g (34,12 mmol) der Titelverbindung aus Beispiel 6b werden in 300 ml Trifluoressigsäure gelöst und 3 Stunden bei Raumtemperatur gerührt. Man dampft zur Trockne ein, nimmt den Rückstand in 80 ml Wasser auf und gibt die Lösung auf eine Säule, gefüllt mit Reillex® 425 PVP. Man eluiert mit Wasser. Die produkthaltigen Fraktionen werden vereinigt und zur Trockne eingedampft, der Rückstand wird aus Methanol/Aceton umkristallisiert.
Ausbeute: 16,22 g (81 % d. Th.) eines farblosen, hygroskopischen Feststoffes
Wassergehalt: 8,4 %

| Elementaranalyse (berechnet auf wasserfreie Substanz): | | | |
|---|---|---|---|
| ber. | C 53,62 | H 6,56 | N 13,03 |
| gef. | C 53,48 | H 6,71 | N 12,87 |

### d) Gadoliniumkomplex des 1-(4-carboxy-2-oxo-1-phenyl-3-azabutyl)-4,7,10-tris(carboxymethyl)-1,4,7,10-tetraazacyclododecans

Zu 15 g (27,90 mmol) der Titelverbindung aus Beispiel 6c, gelöst in 200 ml Wasser, gibt man 5,06 g (13,95 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 18,27 g (92 % d. Th.) eines farblosen kristallinen Pulvers
Wassergehalt: 2,8 %

| Elementaranalyse (berechnet auf wasserfreie Substanz): | | | | |
|---|---|---|---|---|
| ber. | C 41,67 | H 4,66 | N 10,12 | Gd 22,73 |
| gef. | C 41,40 | H 4,80 | N 9,95 | Gd 22,51 |

### Beispiel 7

### a) N-(2-Brompropionyl)-β-alanin

Zu 40 g (448,98 mmol) β-Alanin und 90 g (889,5 mmol) Triethylamin in 500 ml Methylenchlorid tropft man bei 0°C 72,69 g (311,3 mmol) α-Brom-Propionsäurechlorid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 500 ml 5 %iger aqu. Salzsäure extrahiert, über Magnesiumsulfat getrocknet und im Vakuum zur Trockne eingedampft. Der Rückstand wird aus Aceton/Di-isopropylether umkristallisiert.
Ausbeute: 62,37 g (62 % d. Th.)

| Elementaranalyse: | | | | |
|---|---|---|---|---|
| ber. | C 32,16 | H 4,50 | N 6,25 | Br 35,66 |
| gef. | C 32,02 | H 4,65 | N 6,13 | Br 35,74 |

### b) 10-(5-Carboxy-1-methyl-2-oxo-3-azapentyl)-1,4,7,10-tetraazacyclododecan-1,4,7-triessigsäure

Zu 60 g (267,80 mmol) der Titelverbindung aus Beispiel 7a, gelöst in 300 ml Acetonitril/ 200 ml Wasser, gibt man 46,38 g (133,9 mmol) 1,4,7-Tris(carboxymethyl)-1,4,7,10-Tetraazacyclododecan (= DO3A), 129,54 g (937,3 mmol) Kaliumcarbonat und 1 g (6 mmol) Kaliumjodid. Man erhitzt 12 Stunden unter Rückfluß. Es wird im Vakuum zur Trockne eingedampft, der Rückstand in 500 ml Methanol aufgenommen und dann von den Salzen abfiltriert. Das Filtrat wird zur Trockne eingedampft, der Rückstand in 300 ml Wasser aufgenommen und mit 5 N Salzsäure auf pH 1 gestellt. Anschließend wird über eine Säule gefüllt mit Reillex® 425 PVP gereinigt. Man eluiert mit Wasser. Die produkthaltigen Fraktionen werden im Vakuum zur Trockne eingedampft und der Rückstand aus Methanol/Aceton umkristallisiert.
Ausbeute: 19,19 g (27 % d. Th.) eines farblosen Feststoffes
Wassergehalt: 7,8 %

| Elementaranalyse (berechnet auf wasserfreie Substanz): | | | |
|---|---|---|---|
| ber. | C 49,07 | H 7,21 | N 14,31 |
| gef. | C 48,85 | H 7,31 | N 14,19 |

### c) Gadoliniumkomplex der 10-(5-Carboxy-1-methyl-2-oxo-3-azapentyl)-1,4,7,10-tetraazacyclododecan-1,4,7-triessigsäure

Zu 18 g (36,77 mmol) der Titelverbindung aus Beispiel 7b gelöst in 300 ml Wasser, gibt man 6.66 g (18,38 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 21,6 g (89 % d. Th.) eines farblosen kristallinen Pulvers
Wassergehalt: 2,5 %

| Elementaranalyse (berechnet auf wasserfreie Substanz) | | | | |
|---|---|---|---|---|
| ber. | C 37,32 | H 5,01 | N 10,88 | Gd 24,43 |
| gef. | C 37,15 | H 5,21 | N 10,67 | Gd 24,25 |

### Beispiel 8

### a) N-(2-Brompropionyl)-11-aminoundecansäure

Zu 30 g (149 mmol) 11-Aminoundecansäure und 45,24 g (447,1 mmol) Triethylamin in 600 ml Methylenchlorid tropft man bei 0°C 30,65 g (178,8 mmol) α-Brom-Propionsäurechlorid zu. Dabei hält man die Temperatur zwischen 0°C - 5°C. Man gibt 800 ml 5 %ige aqu. Salzsäure zu und trennt die organische Phase ab. Die organische Phase wird noch einmal mit 300 ml 5 %iger aqu. Salzsäure extrahiert, über Magnesiumsulfat getrocknet und im Vakuum zur Trockne eingedampft. Der Rückstand wird aus Aceton/ Di-isopropylether umkristallisiert. Ausbeute: 25,55 g (51 % d. Th.)

| Elementaranalyse: | | | | |
|---|---|---|---|---|
| ber. | C 50,01 | H 7,79 | N 4,17 | Br 23,76 |
| gef. | C 49,82 | H 7,95 | N 4,03 | Br 23,59 |

### b) 10-(13-Carboxy-1-methyl-2-oxo-3-aza-tridecyl)-1,4,7,10-tetraazacyclododecan-1,4,7-triessigsäure

Zu 25 g (74,35 mmol) der Titelverbindung aus Beispiel 8a, gelöst in 250 ml Acetonitril/150 ml Wasser, gibt man 12,88 g (37,18 mmol) 1,4,7-Triscarboxymethyl-1,4,7,10-Tetraazacyclododecan (= DO3A), 35,97 g (260,3 mmol) Kaliumcarbonat und 1 g (6 mmol) Kaliumjodid. Man erhitzt 12 Stunden unter Rückfluß. Es wird im Vakuum zur Trockne eingedampft, der Rückstand in 300 ml Methanol aufgenommen und dann von den Salzen abfiltriert. Das Filtrat wird zur Trockne eingedampft, der Rückstand in 300 ml Wasser aufgenommen und mit 5 N Salzsäure auf pH 1 gestellt. Anschließend wird über eine Säule gefüllt mit Reillex® 425 PVP gereinigt. Man eluiert mit Wasser. Die produkthaltigen Fraktionen werden im Vakuum zur Trockne eingedampft und der Rückstand aus Methanol/Aceton umkristallisiert.
Ausbeute: 6.63 g (27 % d. Th.) eines farblosen Feststoffes
Wassergehalt: 8,9 %

| Elementaranalyse (berechnet auf wasserfreie Substanz): | | | |
|---|---|---|---|
| ber. | C 55,89 | H 8,54 | N 11,64 |
| gef. | C 55,71 | H 8,70 | N 11,57 |

### c) Gadoliniumkomplex der 10-(13-Carboxy-1-methyl-2-oxo-3-aza-tridecyl)-1,4,7,10-tetraazacyclododecan-1,4,7-triessigsäure

Zu 6 g (9,97 mmol) der Titelverbindung aus Beispiel 8b, gelöst in 80 ml Wasser, gibt man 1,81 g (4,98 mmol) Gadoliniumoxid und erwärmt 3 h auf 90°C. Man dampft zur Trockne ein (Vakuum und kristallisiert den Rückstand aus 90 % aqu. 2-Propanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 6,75 g (87 % d. Th.) eines farblosen, kristallinen Pulvers
Wassergehalt: 2,9 %

| Elementaranalyse (berechnet auf wasserfreie Substanz): | | | | |
|---|---|---|---|---|
| ber. | C 44,49 | H 6,40 | N 9,26 | Gd 20,80 |
| gef. | C 44,28 | H 6,55 | N 9,11 | Gd 20,63 |

### Beispiel 9

### a) N-(2-Brompropionyl)-alanin

Zu 30 g (336,7 mmol) Alanin und 102,2 g (1010,2 mmol) Triethylamin in 600 ml Methylenchlorid tropft man bei 0°C 69,26 g (404 mmol) α-Brom-Propionsäurechlorid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 400 ml 5 %iger aqu. Salzsäure extrahiert, über Magnesiumsulfat getrocknet und im Vakuum zur Trockne eingedampft. Der Rückstand wird aus Aceton/ Di-isopropylether umkristallisiert. Ausbeute: 52.05 g (69 % d. Th.)

| Elementaranalyse (berechnet auf wasserfreie Substanz): | | | | |
|---|---|---|---|---|
| ber. | C 32,16 | H 4,50 | N 6,25 | Br 35,66 |
| gef. | C 32,33 | H 4,70 | N 6,13 | Br 35,41 |

### b) 10-(4-Carboxy-1-methyl-2-oxo-3-azapentyl)-1,4,7,10-tetraazacyclododecan-1,4,7-triessigsäure

Zu 50 g (223,2 mmol) der Titelverbindung aus Beispiel 9a, gelöst in 300 ml Acetonitril/200 ml Wasser, gibt man 38,65 g (111,6 mmol) 1,4,7-Triscarboxymethyl-1,4,7,10-Tetraazacyclododecan (= DO3A), 108 g (781,2 mmol) Kaliumcarbonat und 1 g (6 mmol) Kaliumjodid. Man erhitzt 12 Stunden unter Rückfluß. Es wird im. Vakuum zur Trockne eingedampft, der Rückstand in 500 ml Methanol aufgenommen und dann von den Salzen abfiltriert. Das Filtrat wird zur Trockne eingedampft, der Rückstand in 300 ml Wasser aufgenommen und mit 5 N Salzsäure auf pH 1 gestellt. Anschließend wird über eine Säule gefüllt mit Reillex® 425 PVP gereinigt, Man eluiert mit Wasser. Die produkthaltigen Fraktionen werden im Vakuum zur Trockne eingedampft und der Rückstand aus Methanol/Aceton umkristallisiert.
Ausbeute: 17,72 g (30 % d. Th.) eines farblosen Feststoffes
Wassergehalt: 7,5 %

| Elementaranalyse (berechnet auf wasserfreie Substanz): | | | |
|---|---|---|---|
| ber. | C 49,07 | H 7,21 | N 14,31 |
| gef. | C 49,23 | H 7,38 | N 14,15 |

### c) Gadoliniumkomplex der 10-(4-Carboxy-1-methyl-2-oxo-3-azapentyl)-1,4,7,10-tetraazacyclododecan-1,4,7-triessigsäure

Zu 15 g (30,64 mmol) der Titelverbindung aus Beispiel 9b, gelöst in 150 ml Wasser, gibt man 5,55 g (15,32 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 18,22 g (90 % d. Th.) eines farblosen, kristallinen Pulvers
Wassergehalt: 2,6 %

| Elementaranalyse (berechnet auf wasserfreie Substanz): | | | | |
|---|---|---|---|---|
| ber. | C 37,32 | H 5.01 | N 10,88 | Gd 24,43 |
| gef. | C 37,13 | H 5,20 | N 10,61 | Gd 24,41 |

### Beispiel 10

### a) N-(2-Brompropionyl)-valin

Zu 40 g (341,4 mmol) Valin und 103,7 g (1024 mmol) Triethylamin in 600 ml Methylenchlorid tropft man bei 0°C 70,2 g (409,7 mmol) α-Brom-Propionsäurechlorid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 500 ml 5 %iger aqu. Salzsäure extrahiert, über Magnesiumsulfat getrocknet und im Vakuum zur Trockne eingedampft. Der Rückstand wird aus Aceton/ Di-isopropylether umkristallisiert. Ausbeute: 59,39 g (69 % d. Th.)

| Elementaranalyse (berechnet auf wasserfreie Substanz): | | | | |
|---|---|---|---|---|
| ber. | C 38,11 | H 5,60 | N 5,56 | Br 31,69 |
| gef. | C 38,01 | H 5,75 | N 5,41 | Br 31,48 |

### b) 10-(4-Carboxy-1,5-dimethyl-2-oxo-3-azahexyl]-1,4,7,10-tetraazacyclododecan-1,4,7-triessigsäure

Zu 55 g (218,2 mmol) der Titelverbindung aus Beispiel 10a, gelöst in 200 ml Acetonitril/200 ml Wasser, gibt man 37,8 g (109,7 mmol) 1,4,7-Triscarboxymethyl-1,4,7,10-Tetraazacyclododecan (= DO3A), 106,13 g (767,9 mmol) Kaliumcarbonat und 1 g (6 mmol) Kaliumjodid. Man erhitzt 12 Stunden unter Rückfluß. Es wird im Vakuum zur Trockne eingedampft, der Rückstand in 500 ml Methanol aufgenommen und dann von den Salzen abfiltriert. Das Filtrat wird zur Trockne eingedampft, der Rückstand in 300 ml Wasser aufgenommen und mit 5 N Salzsäure auf pH 1 gestellt. Anschließend wird über eine Säule gefüllt mit Reillex® 425 PVP gereinigt. Man eluiert mit Wasser. Die produkthaltigen Fraktionen werden im Vakuum zur Trockne eingedampft und der Rückstand aus Methanol/Aceton umkristallisiert.
Ausbeute: 17.57 g (29 % d. Th.) eines farblosen Feststoffes
Wassergehalt: 6,3 %

| Elementaranalyse (berechnet auf wasserfreie Substanz): | | | | |
|---|---|---|---|---|
| ber. | C 51,05 | H 7,59 | N 13,53 | |
| gef. | C 51,18 | H 7,70 | N 13,39 | |

### c) Gadoliniumkomplex der 10-(4-Carboxy-1,5-dimethy-2-oxo-3-azahexyl]-1,4,7,10-tetraazacyclododecan-1,4,7-triessigsäure

Zu 15 g (28,98 mmol) der Titelverbindung aus Beispiel 10b, gelöst in 150 ml Wasser, gibt man 5,25 g (14,49 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden). Ausbeute: 18,57 g (93 % d. Th.) eines farblosen, kristallinen Pulvers
Wassergehalt: 2,5 %

| Elementaranalyse (berechnet auf wasserfreie Substanz): | | | | |
|---|---|---|---|---|
| ber. | C 39,33 | H 5,40 | N 10,42 | Gd 23,41 |
| gef. | C 39,17 | H 5,55 | N 10,31 | Gd 23,27 |

### Beispiel 11

### a) N-(2-Bromacetyl)-glycin-tert.-butylester

Zu 50 g (296,5 mmol) Glycin-tert.-butylester Hydrochloridsalz und 90 g (889,5 mmol) Triethylamin in 500 ml Methylenchlorid tropft man bei 0°C 77,8 g (385,5 mmol) Brom-Essigsäurebromid zu. Dabei hält man die Temperatur zwischen 0°C - 5°C. Man gibt 1000 ml 5 %ige aqu. Salzsäure zu und trennt die organische Phase ab. Die organische Phase wird noch einmal mit 500 ml 5 %iger aqu. Salzsäure extrahiert, über Magnesiumsulfat getrocknet und im Vakuum zur Trockne eingedampft. Der Rückstand wird aus Diisopropylether/n-Hexan umkristallisiert. Ausbeute: 30,5 g (61 % d. Th.)

| Elementaranalyse: | | | | |
|---|---|---|---|---|
| ber. | C 38,11 | H 5,60 | N 5,56 | Br 31,69 |
| gef. | C 37,92 | H 5,76 | N 5,38 | Br 31,42 |

### b) 10-[4-(tert.Butoxycarbonyl)-2-oxo-3-azabutyl]-1,4,7,10-tetraazacyclododecan-1,4,7-triessigsäure-tri-tert.-butylester

Zu 20,35 g (80,70 mmol) der Titelverbindung aus Beispiel 11a, 25 g (53,8 mmol) 1,4,7-Tris(tert.butoxy-carboxymethyl)-1,4,7,10-tetraazacyclododecan (= DO3A-tri-tert.butylester), 29,74 g (215,8 mmol) Kaliumcarbonat und 1 g (6 mmol) Kaliumjodid gibt man 200 ml Acetonitril und erhitzt 12 Stunden unter Rückfluß. Man filtriert von den Salzen ab und dampft das Filtrat im Vakuum zur Trockne ein. Der Rückstand wird in 800 ml Dichlormethan gelöst und 2 mal mit 200 ml 5 %iger aqu. Natriumcarbonat-Lösung extrahiert. Die organische Phase wird über Magnesiumsulfat getrocknet und eingedampft. Nach Chromatographie an Kieselgel (Laufmittel: Dichlormethan/ Methanol= 20:1) erhält man 25,09 g der Titelverbindung (68 % d. Th.) als farblosen Schaum.

| Elementaranalyse: | | | |
|---|---|---|---|
| ber. | C 59,54 | H 9,26 | N 10,21 |
| gef. | C 59,35 | H 9,42 | N 10,03 |

### c) 10-[4-Carboxy-2-oxo-3-azabutyl]-1,4,7,10-tetraazacyclododecan-1,4,7-triessigsäure

25 g (36,45 mmol) der Titelverbindung aus Beispiel 11b werden in 300 ml Trifluoressigsäure gelöst und 3 Stunden bei Raumtemperatur gerührt. Man dampft zur Trockne ein, nimmt den Rückstand in 80 ml Wasser auf und gibt die Lösung auf eine Säule, gefüllt mit Reillex® 425 PVP. Man eluiert mit Wasser. Die produkthaltigen Fraktionen werden vereinigt und zur Trockne eingedampft, der Rückstand wird aus Methanol/Aceton umkristallisiert.
Ausbeute: 15,24 g (84 % d. Th.) eines farblosen, hygroskopischen Feststoffes
Wassergehalt: 7,3 %

| Elementaranalyse (berechnet auf wasserfreie Substanz): | | | |
|---|---|---|---|
| ber. | C 46,85 | H 6,77 | N 15,18 |
| gef. | C 46,61 | H 6,95 | N 15,02 |

### d) Gadoliniumkomplex der 10-[4-Carboxy-2-oxo-3-azabutyl]-1,4,7,10-tetraazacyclododecan-1,4,7-triessigsäure

Zu 15 g (32.50 mmol) der Titelverbindung aus Beispiel 11c, gelöst in 200 ml Wasser, gibt man 5,86 g (16,25 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 18,92 g (92 % d. Th.) eines farblosen, kristallinen Pulvers
Wassergehalt: 2,7 %

| Elementaranalyse (berechnet auf wasserfreie Substanz): | | | | |
|---|---|---|---|---|
| ber. | C 35,11 | H 4,58 | N 11,37 | Gd 25,54 |
| gef. | C 34,92 | H 4,71 | N 11,14 | Gd 25,33 |

Die nachfolgenden Beispiele 12-37 dienen zur Erläuterung des erfindungsgemäßen Verfahrens.

Beispiele 12-24: In situ Bildung des Metallkomplexcarbonsäure-Gemisches bestehend aus Metallkomplexcarbonsäure und lösungsvermittelndem Stoff:

### Beispiel 12

### a) Bis[2-(benzyloxycarbonylamino)-ethyl]-amin

51,5 g (500 mmol) Diethylentriamin und 139 ml (1 mol) Triethylamin werden in Dichlormethan gelöst und bei -20°C mit 161 g Benzylcyanformiat (Fluka) in Dichlormethan versetzt und anschließend über Nacht bei Raumtemperatur gerührt. Nach Beendigung der Reaktion wird im Abzug eingedampft, der Rückstand in Diethylether aufgenommen, die organische Phase mit Natriumcarbonatlösung gewaschen und mit Natriumsulfat getrocknet. Das Filtrat wird mit Hexan versetzt, der Niederschlag abfiltriert und getrocknet.
Ausbeute: 163,4 g (88 % d. Th.)

| Elementaranalyse: | | | |
|---|---|---|---|
| ber. | C 64,67 | H 6,78 | N 11,31 |
| gef. | C 64,58 | H 6,83 | N 11,28 |

### b) N,N,N',N',N",N"-Hexakis[2-(benzyloxycarbonylamino)-ethyl]-trimesinsäuretriamid

13,27 g (50 mmol) Trimesinsäure-trichlorid (Aldrich) und 34,7 ml (250 mmol) Triethylamin werden in Dimethylformamid (DMF) gelöst und bei 0°C mit 65,0 g (175 mmol) des in Beispiel 12a beschriebenen Amins versetzt und anschließend über Nacht bei Raumtemperatur gerührt.
Die Lösung wird im Vakuum eingedampft und der Rückstand mit Ethylacetat an Kieselgel chromatographiert.
Ausbeute: 39,4 g (62 % d. Th.)

| Elementaranalyse: | | | |
|---|---|---|---|
| ber. | C 65,24 | H 5,95 | N 9,92 |
| gef. | C 65,54 | H 5,95 | N 9,87 |

### c) N^{α}, N^{ε}-Bis(N,N'-dibenzyloxycarbonyl-lysyl)-lysin, geschütztes "Tri-Lysin"

3,6 g (20 mmol) Lysin-Hydrochlorid und 6,95 ml (50 mmol) Triethylamin werden in DMF gelöst, mit 26,8 g (50 mmol) N^{α}, N^{ε}-Dibenzyloxycarbonyl-Lysin-p-nitrophenylester (Bachem) versetzt und 2 Tage bei Raumtemperatur gerührt. Nach Beendigung der Reaktion wird im Vakuum eingedampft, der Rückstand in Ethylacetat aufgenommen und mit verdünnter Salzsäure ausgeschüttelt. Die organische Phase wird mit Natriumsulfat getrocknet, das Lösungsmittel eingedampft und der Rückstand mit Ethylacetat/Ethanol in einem Stufengradienten chromatographiert.
Ausbeute: 10,7 g (57 % d. Th.)

| Elementaranalyse: | | | |
|---|---|---|---|
| ber. | C 63,95 | H 6,65 | N 8,95 |
| gef. | C 63,63 | H 6,69 | N 8,93 |

### d) Voflgeschütztes Benzyloxycarbonyl-24mer-Polyamin auf der Basis des N,N,N',N',N",N"-Hexakis[2-(trilysyl-amino)-ethyl]-trimesinsäuretriamids

1,27 g (1 mmol) des im Beispiel 12b beschriebenen Hexa-Benzyloxycarbonylamins werden in Eisessig gelöst und unter Rühren mit 33 prozentigem Bromwasserstoff in Eisessig versetzt. Nach 60 Minuten wird mit Diethylether die begonnene Fällung vervollständigt, das entstandene Hexa-amin-hydrobromid mit Ether gewaschen, im Vakuum getrocknet und ohne weitere Reinigung in die weiter unten beschriebene Reaktion eingesetzt.
Ausbeute: 0,95 g (quantitativ)

7,0 g (7,5 mmol) des in Beispiel 12c beschriebenen geschützten "Tri-Lysins", 1,2 g (7,5 mmol) 1-Hydroxybenzotriazol und 2,4 g (7,5 mmol) 2-(1H-Benzotriazol-1-yl)-1,1,3,3-tetramethyluronium tetratluorborat (TBTU; Peboc Limited, UK) werden in DMF gelöst und 15 Minuten gerührt. Diese Lösung wird anschließend mit 5,16 ml (30 mmol) N-Ethyldiisopropylamin und mit 0,95 g (1 mmol) des oben beschriebenen Hexa-amin-hydrobromids versetzt und über Nacht bei Raumtemperatur gerührt. Nach Beendigung der Reaktion wird im Vakuum eingedampft und der Rückstand mit Ethylacetat/Ethanol (2:1) an Kieselgel chromatographiert.
Ausbeute: 4,55 g (76 % d. Th.)

| Elementaranalyse: | | | |
|---|---|---|---|
| ber. | C 64,35 | H 6,71 | N 10,52 |
| gef. | C 64,08 | H 6,57 | N 10.29 |

### e) 24mer-Polyamin auf der Basis des N,N,N',N',N",N"-Hexakis[2-(trilysyl-amino)-ethyl]-trimesinsäuretriamids, Tetracosahydrochlorid

5,99 g (1 mmol) des im vorstehenden Beispiel 12d beschriebenen 24-mer Benzyloxycarbonylamins werden in 500 ml Methanol gelöst, unter Stickstoff mit 1 g Palladium auf Aktivkohle (10 %) und mit 24 ml (24 mmol) 1 N HCl versetzt und mit Wasserstoff hydriert. Nach Beendigung der Reaktion wird vom Katalysator abfiltriert und das Filtrat zur Trockne eingedampft.
Ausbeute: 3,65 g (quantitativ). Das Tetracosahydrochlorid löst sich klar in Wasser

| Elementaranalyse: | | | | |
|---|---|---|---|---|
| ber. | C 42,48 | H 7,71 | N 17,28 | Cl 23,33 |
| gef. | C 42,20 | H 7,94 | N 17,09 | Cl 25,06 |

### f) Tetracosakis-Amid-Konjugat des 24mer-Polyamins auf der Basis des N,N,N',N',N",N"-Hexakis[2-(trilysyl-amino)-ethyl]-trimesinsäuretriamids mit dem Gadoliniumkomplex der 10-(4-Carboxy-1-methyl-2-oxo-3-azabutyl)-1,4,7,10-tetraazacyclododecan-1,4,7-triessigsäure

31,74 g (50,4 mmoi, dreifacher Überschuß) des in Beispiel 1f beschriebenen Gd-Komplexes der 10-(4-Carboxy-1-methyl-2-oxo-3-azabutyl)-1,4,7,10-tetraazacyclododecan-1,4,7-triessigsäure,5,19 g Natriumbromid (50.4 mmol) und 8,7 g (75,6 mmol) N-Hydroxysuccinimid werden in 250 ml Dimethylsulfoxid bei 50°C gelöst. Nach Abkühlen auf Raumtemperatur werden 15,6 g (75,6 mmol) N,N'-Dicyclohexylcarbodiimid zugesetzt und 60 Minuten voraktiviert. Zu der so hergestellten N-Hydroxysuccinimidester-Lösung gibt man eine Lösung von 2,55 g (0,7 mmol) des im Beispiel 12e beschriebenen Tetracosahydrochlorids und 8,5 g Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet.
Ausbeute: 11,8 g (88 % d. Th.)
H₂O-Gehalt (Karl-Fischer): 9,0 %
Gd-Bestimmung (AAS): 19,6 %
MALDI-TOF-Massenspektrum: 17,476 (M+Na⁺)

| Elementaranalyse (berechnet auf wasserfreie Substanz): | | | | |
|---|---|---|---|---|
| ber. | C 40,26 | H 5,35 | N 13,24 | Gd 21,62 |
| gef. | C 40,04 | H 5,59 | N 13,47 | Gd 20,88 |

### Beispiel 13

### a) 1,4,7-Tris[N2,N6-bis(benzyloxycarbonyl)-lysyl]-1,4,7,10-tetraazacyclododecan

49,07 g (95,9 mmol) Di-Z-Lysin-N-hydroxysuccinimidester und 5 g (29 mmol) Cyclen (= 1,4,7,10-tetraazacyclododecan) werden in einer Mischung aus 200 ml Toluol/100 ml Dioxan gelöst. Man setzt 9,7 g (95,9 mmol) Triethylamin zu und erhitzt 12 Stunden auf 70°C. Man dampft zur Trockne ein, nimmt den Rückstand in 600 ml Dichlormethan auf und extrahiert 3 mal mit je 200 ml 5 %iger aqu. Kaliumcarbonat-Lösung. Die organische Phase wird über Magnesiumsulfat getrocknet und im Vakuum zur Trockne eingedampft. Der Rückstand wird an Kieselgel chromatographiert (Laufmittel: Ethylacetat/Ethanol= 15:1). Ausbeute: 29,61 g (75 % d. Th.) eines farblosen Feststoffes

| Elementaranalyse: | | | |
|---|---|---|---|
| ber. | C 65,28 | H 6,81 | N 10,29 |
| gef. | C 65,41 | H 6,97 | N 10,10 |

### b) 1-(4-Carboxybutyryl)-4,7,10-tris(N2,N6-bis(benzyloxycarbonyl)-lysyl]-1,4,7,10-tetraazacyclododecan

Zu 28 g (20,56 mmol) der Titelverbindung aus Beispiel 13a (gelöst in 200 ml Tetrahydrofuran) gibt man 3,5 g (30,8 mmol) Glutarsäureanhydrid (Fluka) und 6,24 g (61,72 mmol) Triethylamin. Man erwärmt 6 Stunden auf 50°C. Die Lösung wird im Vakuum zur Trockne eingedampft, mit 300 ml Dichlormethan aufgenommen und zweimal mit je 150 ml 5 %iger aqu. Salzsäure extrahiert. Die organische Phase wird über Magnesiumsulfat getrocknet, im Vakuum zur Trockne eingedampft und der Rückstand an Kieselgel chromatographiert (Laufmittel:
Dichlormethan/Methanol = 20:1).
Ausbeute: 27,65 g (91 % d. Th.) eines farblosen Feststoffes

| Elementaranalyse: | | | |
|---|---|---|---|
| ber. | C 64,30 | H 6,70 | N 9,49 |
| gef. | C 64,18 | H 6,75 | N 9,61 |

### c) 1-(4-Nitrophenoxy)-glutaryl)-4,7,10-tris[N2,N6-bis(benzyloxycarbonyl)-lysyl]-1,4,7,10-tetraazacyclododecan

14,76 g (10 mmol) der im Beispiel 13b beschriebenen Carbonsäure, gelöst in 150 ml Dichlormethan, werden zunächst mit 1,53 g (11 mmol) 4-Nitrophenol und anschließend bei 0°C mit 2,27 g (11 mmol) Dicyclohexylcarbodiimid versetzt. Nach Rühren über Nacht bei Raumtemperatur wird vom Dicyclohexylharnstoff abgesaugt, das Filtrat eingeengt und aus Isopropanol umgefällt, Vom ölig anfallenden Produkt wird die Mutterlauge abdekantiert, das Öl in Dichlormethan aufgenommen und im Vakuum eingeengt. Man erhält 15,4 g (96,3 %) schaumig erstarrten Feststoff.

| Elementaranalyse: | | | |
|---|---|---|---|
| ber. | C 63,94 | H 6,38 | N 9,65 |
| gef. | C 63,69 | H 6,31 | N 9,88 |

### d) Vollgeschütztes Benzyloxycarbonyl-36mer-Polyamin, aufgebaut aus N,N,N',N',N",N"-Hexakis(2-aminoethyl)-trimesinsäuretriamid-Core und sechs im Beispiel 13b beschriebenen amingeschützten Hexaamin-monocarbonsäuren

1,27 g (1 mmol) des im Beispiel 12b beschriebenen Hexakis-Benzyloxycarbonyl-amins werden in Eisessig gelöst und unter Rühren mit 33 prozentigem Bromwasserstoff in Eisessig versetzt. Nach 60 Minuten wird mit Diethylether die begonnene Fällung vervollständigt, das entstandene Hexa-amin-hexa-hydrobromid mit Ether gewaschen, im Vakuum getrocknet und ohne weitere Reinigung weiter umgesetzt.
Ausbeute: 0,95 g (quantitativ)
Anschließend wird das Hexa-amin-hexa-hydrobromid in 150 ml DMF gelöst, mit 15,99 g (10 mmol) des in Beispiel 13c beschriebenen 4-Nitrophenylaktivesters und mit 4,05 g (40 mmol) Triethylamin versetzt, über Nacht bei Raumtemperatur gerührt und schließlich im Vakuum zur Trockne eingedampft. Der Rückstand wird in Ethylacetat aufgenommen und nacheinander mit Wasser, verd. Natronlauge und gesättigter NaCl-Lösung gewaschen. Die organische Phase wird über Natriumsulfat getrocknet und das Filtrat zur Trockne eingedampft und der Rückstand an Kieselgel (Laufmittel: Dichlormethan/Methanol 18:2) chromatographiert. Ausbeute: 6,55 g (71 % d. Th.) eines farblosen Feststoffes

| Elementaranalyse: | | | |
|---|---|---|---|
| ber. | C 64,54 | H 6,73 | N 10,49 |
| gef. | C 64,37 | H 6,91 | N 10,74 |

### MALDI-TOF-Massenspektrum: Molpeak bei 9235 (M + Na⁺)

### e) Hexatriacontakis-Amid-Konjugat des 36mer-Polyamins aus Beispiel 13d mit dem Dysprosium-Komplex der 10-(4-Carboxy-1-methyl-2-oxo-3-azabutyl)-1,4,7,10-tetraazacyclododecan-1,4,7-triessigsäure

1,84 (0,2 mmol) des im Beispiel 13d beschriebenen 36mer-Benzyloxycarbonylamins werden in Eisessig gelöst und unter Rühren mit 33 prozentigem Bromwasserstoff in Eisessig versetzt. Nach 5 Stunden wird mit Diethylether die begonnene Fällung vervollständigt, das entstandene 36mer-Amin-hydrobromid mit Ether gewaschen, im Vakuum getrocknet und ohne weitere Reinigung in die weiter unten beschriebene Reaktion eingesetzt.
Ausbeute: 1,5 g (quantitativ)
13,6 g (21,6 mmol, dreifacher Überschuß) des in Beispiel 2 beschriebenen Dysprosium-Komplexes der 10-(4-Carboxy-1-methyl-2-oxo-3-azabutyl)-1,4,7,10-tetraazacyclododecan-1,4,7-triessigsäure und 3,73 g (32,4 mmol) N-Hydroxysuccinimid) werden in 100 ml Dimethylsulfoxid bei 90°C gelöst. Nach Abkühlen auf Raumtemperatur werden 6,7 g (32,4 mmol) N,N'-Dicyclohexylcarbodiimid zugesetzt und 60 Minuten voraktiviert. Zu der so hergestellten N-Hydroxysuccinimidester-Lösung gibt man eine Lösung von 1,5 g (0,2 mmol) des oben beschriebenen Hexatriaconta-hydrobromids und 3,5 g Triethylamin in 10 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON®YM-3 Ultrafiltrationsmembran (cut off 3000 Da) entsalzt und von niedermolekularen Bestandteilen gereinigt. Das Retentat wird anschließend gefriergetrocknet. Ausbeute: 4,7 g (79 % d. Th.)
H₂O-Gehalt (Karl-Fischer): 10,5 %
Dy-Bestimmung (AAS): 19,7 %
MALDI-TOF-Massenspektrum: 26,616 (M + Na⁺)

| Elementaranalyse (berechnet auf wasserfreie Substanz): | | | | |
|---|---|---|---|---|
| ber. | C 40,24 | H 5,35 | N 13,11 | Dy 22,00 |
| gef. | C 39,97 | H 5,46 | N 12,90 | Dy 21,29 |

### Beispiel 14

### Polyamid-Konjugat von poly-Lysin mit dem Gadoliniumkomplex der 10-(4-Carboxy-1-methyl-2-oxo-3-azabutyl)-1,4,7,10-tetraazacyclododecan-1,4,7-triessigsäure

3,15 g (5 mmol) des in Beispiel 1f beschriebenen Gd-Komplexes und 0,58 g (5 mmol) N-Hydroxysuccinimid werden in 25 ml Dimethylsulfoxid unter Erwärmen gelöst. Nach Abkühlen auf Raumtemperatur werden 1,03 g (5 mmol) N,N'-Dicyclohexylcarbodiimid zugesetzt und 60 Minuten gerührt. Zu der so hergestellten N-Hydroxysuccinimidester-Lösung gibt man eine Lösung von 523 mg (2,5 mmol) poly-Lysin-Hydrobromid (Sigma) und 506 mg (5 mmol) Triethylamin in 5 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen von unlöslichen Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet.
Ausbeute: 1,8 g (82 % d. Th.)
H₂O-Gehalt (Karl-Fischer): 10,2 %
Gd-Bestimmung (AAS): 19,0 %

| Elementaranalyse (berechnet auf wasserfreie Substanz): | | | | |
|---|---|---|---|---|
| ber. | C 40,58 | H 5,45 | N 13,25 | Gd 21,25 |
| gef. | C 40,81 | H 5,21 | N 13,44 | Gd 20,87 |

### Beispiel 15

### Dotriacontakis-Amid-Konjugat aus dem 32mer-Dendrimer-amin "DAB(PA)₄(PA)₈(PA)₁₆(PA)₃₂" und dem Dysprosiumkomplex der 10-(4-Carboxy-1-ethyl-2-oxo-3-azabutyl)-1,4,7,10-tetraazacyclododecan-1,4,7-triessigsäure

3,25 g (5 mmol) des im Beispiel 5 beschriebenen Dy-Komplexes der 10-(4-Carboxy-1-ethyl-2-oxo-3-azabutyl)-1,4,7,10-tetraazacyclododecan-1,4,7-triessigsäure und 0,58 g (5 mmol) N-Hydroxysuccinimid werden in 25 ml Dimethylsulfoxid unter Erwärmen gelöst. Nach Abkühlen auf Raumtemperatur werden 1,03 g (5 mmol) N,N'-Dicyclohexylcarbodiimid zugesetzt und eine Stunde gerührt. Zu der so hergestellten N-Hydroxysuccinimidester-Lösung gibt man 275 mg (0,078 mmol) des in Beispiel VIII der WO 93/14147 beschriebenen 32mer-Dendrimer-Amins und 506 mg (5 mmol) Triethylamin in 5 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen von unlöslichen Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet. Ausbeute: 1,62 g (82,7 % d. Th.)
H₂O-Gehalt (Karl-Fischer): 7,3 %
Dy-Bestimmung (AAS): 20,5 %

| Elementaranalyse (berechnet auf wasserfreie Substanz): | | | | |
|---|---|---|---|---|
| ber. | C 40,90 | H 5,76 | N 13,37 | Dy 22,36 |
| gef. | C 40,55 | H 6,07 | N 13,69 | Dy 21,83 |

### Beispiel 16

### Amid-Konjugat von Daunomycin mit dem Gadoliniumkomplex von 1-[1-Methyl-2-oxo-3-aza-4-carboxybutyl]-4,7,10-tris(carboxymethyl)-1,4,7,10-tetraazacyclododecan

1 g (1,588 mmol) des Gadoliniumkomplexes von 1-[1-Methyl-2-oxo-3-aza-4-carboxybutyl]-4,7,10-tris(carboxymethyl)-1,4,7,10-tetraazacyclododecan, 134,6 mg (3,176 mmol) Lithiumchlorid und 365,8 mg (3,176 mmol) N-Hydroxysuccinimid werden in 10 ml Dimethylsulfoxid unter leichtem Erwärmen gelöst und anschließend auf 10°C abgekühlt. Man gibt bei 10°C 393 mg (1,906 mmol) Dicyclohexylcarbodiimid hinzu und rührt 45 Minuten bei Raumtemperatur. Zu der so hergestellten N-Hydroxysuccinimidesterlösung gibt man 812,3 mg (1,588 mmol) Daunomycin gelöst in 5 ml Dimethylsulfoxid zu, anschließend 321,4 mg (3,176 mmol) Triethylamin. Man rührt über Nacht bei Raumtemperatur. Zu der erhaltenen Suspension tropft man 200 ml Aceton, filtriert den Niederschlag ab und wäscht ihn 2 mal mit etwas Aceton nach. Der Rückstand wird säulenchromatographisch gereinigt (RP18/Laufmittel: Gradient aus Wasser/Acetonitril/Tetrahydrofuran).
Ausbeute: 1,76 g (85 % d. Th.) farbloses, flockiges Pulver nach Gefriertrocknung Wassergehalt: 6,3 %

| Elementaranalyse (berechnet auf wasserfreie Substanz): | | | | |
|---|---|---|---|---|
| ber. | C 49,19 | H 5,11 | N 7,48 | Gd 14,00 |
| gef. | C 49,03 | H 5,27 | N 7,31 | Gd 13,82 |

### Beispiel 17

### Amid-Konjugat von Adriamycin mit dem Gadoliniumkomplex von 1-[1-Methyl-2-oxo-3-aza-4-carboxybutyl]-4,7,10-tris(carboxymethyl)-1,4,7,10-tetraazacyclododecan

1 g (1,588 mmol) des Gadoliniumkomplexes von 1-[1-Methyl-2-oxo-3-aza-4-carboxybutyl]-4,7,10-tris(carboxymethyl)-1,4,7,10-tetraazacyclododecan, 134,6 mg (3,176 mmol) Lithiumchlorid und 365,8 mg (3,176 mmol) N-Hydroxysuccinimid werden in 10 ml Dimethylsulfoxid unter leichtem Erwärmen gelöst und anschließend auf 10°C abgekühlt. Man gibt bei 10°C 393 mg (1,906 mmol) Dicyclohexylcarbodiimid hinzu und rührt 45 Minuten bei Raumtemperatur. Zu der so hergestellten N-Hydroxysuccinimidesterlösung gibt man 812,3 mg (1,588 mmol) Adriamycin gelöst in 5 ml Dimethylsulfoxid zu. anschließend 321,4 mg (3,176 mmol) Triethylamin. Man rührt über Nacht bei Raumtemperatur. Zu der erhaltenen Suspension tropft man 200 ml Aceton, filtriert den Niederschlag ab und wäscht ihn 2 mal mit etwas Aceton nach. Der Rückstand wird säulenchromatographisch gereinigt (RP18/Laufmittel: Gradient aus Wasser/Acetonitril/Tetrahydrofuran).
Ausbeute: 1,84 g (87 % d. Th.) farbloses, flockiges Pulver nach Gefriertrocknung
Wassergehalt: 7,2 %

| Elementaranalyse (berechnet auf wasserfreie Substanz): | | | | |
|---|---|---|---|---|
| ber. | C 48,50 | H 5,04 | N 7,38 | Gd 13,80 |
| gef. | C 48,37 | H 5,18 | N 7,21 | Gd 13,60 |

### Beispiel 18

### Polyamid-Konjugat von Human-albumin mit dem Gadoliniumkomplex der 10-(5-Carboxy-1-methyl-2-oxo-3-azapentyl)-1,4,7,10-tetraazacyclododecan-1,4,7-triessigsäure

3,22 g (5 mmol) des Gadoliniumkomplexes der 10-(5-Carboxy-1-methyl-2-oxo-3-azapentyl)-1,4,7,10-tetraazacyclododecan-1,4,7-triessigsäure (Beispiel 7c), 868 mg (10 mmol) Lithiumbromid und 1,15g (10 mmol) N-Hydroxysuccinimid werden in 30 ml Dimethylsulfoxid unter leichtem Erwärmen gelöst und anschließend auf 10°C abgekühlt. Man gibt bei 10°C 1,24 g (6 mmol) Dicyclohexylcarbodiimid hinzu und rührt 8 Stunden bei Raumtemperatur. Die so hergestellte N-Hydroxysuccinimidesterlösung wird erneut auf 10°C gekühlt und zu einer Suspension von 6,6 g Humanserumalbumin (Sigma), die durch Zugabe von 5 ml 1 N Natronlauge auf ca.pH 10 eingestellt ist, bei 10°C zugegeben und über Nacht bei dieser Temperatur gerührt. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-10 Ultrafiltrationsmembran (cut off 10.000 Da) entsalzt und von niedermolekularen Bestandteilen gereinigt. Das Retentat wird anschließend gefriergetrocknet. Man erhält ein farbloses Pulver.
Ausbeute: 7,9 g (89 % d. Th., bezogen auf ein Konjugat mit 21 Gd-Komplexen)
H₂O-Gehalt (Karl-Fischer): 10,12 %
Gd-Bestimmung (AAS): 3,77 %

| Elementaranalyse (berechnet auf wasserfreie Substanz eines HSA(Gd)₂₁-Konjugats): | |
|---|---|
| ber. | Gd 4,15 |
| gef. | Gd 4,09 |

### Beispiel 19

### Hexaamid-Konjugat von 6,6',6'',6''',6'''',6'''''-Hexaamino-6,6',6'',6''',6'''',6'''''-hexadeoxy-α-cyclodextrin mit dem Gadoliniumkomplex der 10-(13-Carboxy-1-methyl-2-oxo-3-azatridecyl)-1,4,7,10-tetraazacyclododecan-1,4,7-triessigsäure

3,78 g (5 mmol) des Gadoliniumkomplexes der 10-(5-Carboxy-1-methyl-2-oxo-3-azatridecyl)-1,4,7,10-tetraazacyclododecan-1,4,7-triessigsäure (Beispiel 8c), 868 mg (10 mmol) Lithiumbromid und 1,15g (10 mmol) N-Hydroxysuccinimid werden in 30 ml Dimethylsulfoxid unter leichtem Erwärmen gelöst und anschließend auf 10°C abgekühlt. Man gibt bei 10°C 1,24 g (6 mmol) Dicyclohexylcarbodiimid hinzu und rührt 8 Stunden bei Raumtemperatur. Die so hergestellte N-Hydroxysuccinimidesterlösung wird zu einer Suspension von 315 mg (0,25 mmol) 6,6',6",6"',6"",6""'-Hexaamino-6,6',6",6"',6"",6""'-hexadeoxy-α-cyclodextrin [J.Boger.R.J.Corcoran und J.-M.Lehn, Helv. Chim. Acta 61, 2190 -2218 (1978)] in Dimethylsulfoxid zugegeben und über Nacht bei Raumtemperatur gerührt. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet. Man erhält ein farbloses Pulver. Ausbeute: 1,34 g (89,9 % d. Th.)
H₂O-Gehalt (Karl-Fischer): 9,5 %
Gd-Bestimmung (AAS): 16,4 %

| Elementaranalyse (berechnet auf wasserfreie Substanz): | | | | |
|---|---|---|---|---|
| ber. | C 45,42 | H 6,39 | N 9,35 | Gd 17,49 |
| gef. | C 45,25 | H 6,14 | N 9,69 | Gd 17,04 |

### Beispiel 20

### Amid-Konjugat von 3'-Amino-3'-deoxyguanosin mit dem Gadoliniumkomplex von 1-[1-Methyl-2-oxo-3-aza-4-carboxybutyl]-4,7,10-tris(carboxymethyl)-1,4,7,10-tetraazacyclododecan

1 g (1,588 mmol) des Gadoliniumkomplexes von 1-[1-Methyl-2-oxo-3-aza-4-carboxybutyl]-4,7,10-tris(carboxymethyl)-1,4,7,10-tetraazacyclododecan, 476 mg (3,176 mmol) Natriumjodid und 365,8 mg (3,176 mmol) N-Hydroxysuccinimid werden in 10 ml Dimethylsulfoxid unter leichtem Erwärmen gelöst und anschließend auf 10°C abgekühlt. Man gibt bei 10°C 393 mg (1,906 mmol) Dicyclohexylcarbodiimid hinzu und rührt 45 Minuten bei Raumtemperatur. Zu der so hergestellten N-Hydroxysuccinimidester-Lösung gibt man 422,8 mg (1,588 mmol) 3'-Amino-3'-deoxyguanosin, gelöst in 5 ml Dimethylsulfoxid zu, anschließend 321,4 mg (3,176 mmol) Triethylamin. Man rührt über Nacht bei Raumtemperatur. Zu dieser Lösung tropft man 200 ml Aceton, filtriert den Niederschlag ab und wäscht ihn 2 mal mit etwas Aceton nach. Der Rückstand wird säulenchromatographisch gereinigt (RP18/Laufmittel: Gradient aus Wasser/Acetonitril/Tetrahydrofuran).
Ausbeute: 1,30 g (88 % d. Th.) farbloses, flockiges Pulver nach Gefriertrocknung Wassergehalt: 5,3 %

| Elementaranalyse (berechnet auf wasserfreie Substanz): | | | | |
|---|---|---|---|---|
| ber. | C 39,67 | H 4,82 | N 17,55 | Gd 17,91 |
| gef. | C 39,54 | H 4,97 | N 17,43 | Gd 17,82 |

### Beispiel 21

### Amid-Konjugat von Dehydroabiethylamin mit dem Gadoliniumkomplex von 1-[1-Methyl-2-oxo-3-aza-4-carboxybutyl]-4,7,10-tris(carboxymethyl)-1,4,7,10-tetraazacyclododecan

1 g (1,588 mmol) des Gadoliniumkomplexes von 1-[1-Methyl-2-oxo-3-aza-4-carboxybutyl]-4,7,10-tris(carboxymethyl)-1,4,7,10-tetraazacyclododecan, 134,6 mg (3,176 mmol) Lithiumchlorid und 442 mg (3,176 mmol) 4-Nitrophenol werden in 10 ml Dimethylsulfoxid unter leichtem Erwärmen gelöst und anschließend auf 10°C abgekühlt. Man gibt bei 10°C 393 mg (1,906 mmol) Dicyclohexylcarbodiimid hinzu und rührt 45 Minuten bei Raumtemperatur. Zu der so hergestellten Lösung gibt man 422,8 mg (1,588 mmol) Dehydroabiethylamin, gelöst in 5 ml Dimethylsulfoxid zu. anschließend 321,4 mg (3,176 mmol) Triethylamin. Man rührt über Nacht bei Raumtemperatur. Zu dieser Lösung tropft man 100 ml Aceton, filtriert den Niederschlag ab und wäscht ihn 2 mal mit etwas Aceton nach. Der Rückstand wird säulenchromatographisch gereinigt (RP18/Laufmittel: Gradient aus Wasser/ Acetonitril/Tetrahydrofuran).
Ausbeute: 1,36 g (91 % d. Th.) glasiger Feststoff nach Gefriertrocknung
Wassergehalt: 4,5 %

| Elementaranalyse (berechnet auf wasserfreie Substanz): | | | | |
|---|---|---|---|---|
| ber. | C 52,21 | H 6,63 | N 9,37 | Gd 17,53 |
| gef. | C 52,10 | H 6,70 | N 9,25 | Gd 17,41 |

### Beispiel 22

### Amid-Konjugat von Ampicillin mit dem Gadoliniumkomplex von 1-[1-Methyl-2-oxo-3-aza-4-carboxybutyl]-4,7,10-tris(carboxymethyl)-1,4,7,10-tetraazacyclododecan

1 g (1,588 mmol) des Gadoliniumkomplexes von 1-[1-Methyl-2-oxo-3-aza-4-carboxybutyl]-4,7,10-tris(carboxymethyl)-1,4,7,10-tetraazacyclododecan, 134,6 mg (3,176 mmol) Lithiumchlorid und 442 mg (3,176 mmol) 4-Nitrophenol werden in 10 ml Dimethylsulfoxid unter leichtem Erwärmen gelöst und anschließend auf 10°C abgekühlt. Man gibt bei 10°C 393 g (1,906 mmol) Dicyclohexylcarbodiimid hinzu und rührt 45 Minuten bei Raumtemperatur. Zu der so hergestellten Suspension gibt man 554,9 mg (1,588 mmol) Ampicillin, gelöst in 5 ml Dimethylsulfoxid zu, anschließend 482 mg (4,764 mmol) Triethylamin. Man rührt über Nacht bei Raumtemperatur. Zu dieser Lösung tropft man 100 ml Aceton, filtriert den Niederschlag ab und wäscht ihn 2 mal mit etwas Aceton nach. Der Rückstand wird säulenchromatographisch gereinigt (RP18/Laufmittel: Gradient aus Wasser/ Acetonitril/Tetrahydrofuran / 0,5 *%* Trifluoressigsäure).
Ausbeute: 1,46 g (87 % d. Th.) farbloses, flockiges Pulver nach Gefriertrocknung
Wassergehalt: 8,1 %

| Elementaranalyse (berechnet auf wasserfreie Substanz): | | | | | |
|---|---|---|---|---|---|
| ber. | C 45,76 | H 4,88 | N 10,10 | S 3,30 | Gd 16,19 |
| gef. | C 45,65 | H 5,01 | N 10,02 | S 3,19 | Gd 16,03 |

### Beispiel 23

### Amid-Konjugat von Pentapeptid NH₂-Val-Leu-Phe-Phe-Ala-OH mit dem Gadoliniumkomplex von 1-[1-Methyl-2-oxo-3-aza-4-carboxybutyl]-4,7,10-tris(carboxymethyl)-1,4,7,10-tetraazacyclododecan

1 g (1,588 mmol) des Gadoliniumkomplexes von 1-[1-Methyl-2-oxo-3-aza-4-carboxybutyl]-4,7,10-tris(carboxymethyl)-1,4,7,10-tetraazacyclododecan, 134,6 mg (3,176 mmol) Lithiumchlorid und 365,8 mg (3,176 mmol) N-Hydroxysuccinimid werden in 10 ml Dimethylsulfoxid unter leichtem Erwärmen gelöst und anschließend auf 10°C abgekühlt. Man gibt bei 10°C 393 mg (1,906 mmol) Dicyclohexylcarbodiimid hinzu und rührt 45 Minuten bei Raumtemperatur. Zu der so hergestellten N-Hydroxysuccinimidester-Lösung gibt man 946 mg (1,588 mmol) des Pentapeptids NH₂-Val-Leu-Phe-Phe-Ala-OH (hergestellt nach der Festphasentechnik), gelöst in 5 ml Dimethylsulfoxid zu, anschließend 321,4 mg (3,176 mmol) Triethylamin. Man rührt über Nacht bei Raumtemperatur. Zu dieser Lösung tropft man 200 ml Aceton, filtriert den Niederschlag ab und wäscht ihn 2 mal mit etwas Aceton nach. Der Rückstand wird säulenchromatographisch gereinigt (RP18/Laufmittel: Gradient aus Wasser/Acetonitril/Tetrahydrofuran).
Ausbeute: 1,74g (85 % d. Th.) farbloses, flockiges Pulver nach Gefriertrocknung
Wassergehalt: 6,5 %

| Elementaranalyse (berechnet auf wasserfreie Substanz): | | | | |
|---|---|---|---|---|
| ber. | C 50,73 | H 6,09 | N 11,60 | Gd 13,02 |
| gef. | C 50,58 | H 6,21 | N 11,48 | Gd 12,86 |

### Beispiel 24

### Amid-Konjugat von N-Methylglucosamin mit dem Gadoliniumkomplex von 1-[1-Methyl-2-oxo-3-aza-4-carboxybutyl]-4,7,10-tris(carboxymethyl)-1,4,7,10-tetraazacyclododecan

1 g (1,588 mmol) des Gadoliniumkomplexes von 1-[1-Methyl-2-oxo-3-aza-4-carboxybutyl]-4,7,10-tris(carboxymethyl)-1,4,7,10-tetraazacyclododecan, 134,6 mg (3,176 mmol) Lithiumchlorid werden in 10 ml Dimethylsulfoxid unter leichtem Erwärmen gelöst und anschließend auf 10°C abgekühlt. Man gibt bei 10°C 310 mg (1.588 mmol) D(-)-N-Methylglucosamin und 785,4 mg (3,176 mmol) 2-Ethoxy-1-ethoxycarbonyl-1,2-dihydrochinolin (EEDQ) zu. Man rührt über Nacht bei Raumtemperatur. Zu dieser Lösung tropft man 200 ml Aceton, filtriert den Niederschlag ab und wäscht ihn 2 mal mit etwas Aceton nach. Der Rückstand wird säulenchromatographisch gereinigt (RP18/Laufmittel: Gradient aus Wasser/Acetonitril/Tetrahydrofuran).
Ausbeute: 1,25 g (91 % d. Th.) farbloses, flockiges Pulver nach Gefriertrocknung Wassergehalt: 6,7 %

| Elementaranalyse (berechnet auf wasserfreie Substanz): | | | | |
|---|---|---|---|---|
| ber. | C 38,70 | H 5,62 | N 10,41 | Gd 19,49 |
| gef. | C 38,60 | H 5,73 | N 10,28 | Gd 19,35 |

### Beispiele 25-37: Isolierung des Metallkomplexcarbonsäure-Gemisches bestehend aus Metallkomplexcarbonsäure und lösungsvermittelndem Stoff:

### Beispiel 25

### a) Gemisch bestehend aus dem Gadoliniumkomplex der 10-(4-Carboxy-1-methyl-2-oxo-3-azabutyl)-1,4,7,10-tetraazacyclododecan-1,4,7-triessigsäure und 2 Moläquivalenten Lithiumchlorid

31,74 g (50,4 mmol) des in Beispiel 1f beschriebenen Gd-Komplexes der 10-(4-Carboxy-1-methyl-2-oxo-3-azabutyl)-1,4,7,10-tetraazacyclododecan-1,4,7-triessigsäure werden in 200 ml Wasser gelöst und mit 4,27 g (100,8 mmol) Lithiumchlorid versetzt.Die klare Lösung wird anschließend gefriergetrocknet und schließlich über Nacht bei 100°C im Vakuum nachgetrocknet. Man erhält ein farbloses Pulver.
Ausbeute: 36,0 g (quantitativ)
H₂O-Gehalt (Karl-Fischer): 3,5 %
Gd-Bestimmung (AAS): 21,2 %

| Elementaranalyse (berechnet auf wasserfreie Substanz): | | | | | | |
|---|---|---|---|---|---|---|
| ber. | C 31,94 | H 4,23 | N 9,80 | Gd 22,01 | Li 1,94 | Cl 9,92 |
| gef. | C 31,72 | H 4,44 | N 9,57 | Gd 21,69 | Li 1,98 | Cl 9,70 |

### b) Tetracosakis-Amid-Konjugat des 24mer-Polyamins auf der Basis des N,N,N',N',N",N"-Hexakis[2-(trilysyl-amino)-ethyl]-trimesinsäuretriamids mit dem Gadoliniumkomplex der 10-(4-Carboxy-1-methyl-2-oxo-3-azabutyl)-1,4,7,10-tetraazacyclododecan-1,4,7-triessigsäure

36,0 g (50,4 mmol. dreifacher Überschuß) des im vorstehenden Beispiel 25a beschriebenen Lithium-Mischsalzes mit dem Gd-Komplex der 10-(4-Carboxy-1-methyl-2-oxo-3-azabutyl)-1,4,7,10-tetraazacyclododecan-1,4,7-triessigsäure und 8,7 g (75,6 mmol) N-Hydroxysuccinimid werden in 250 ml Dimethylsulfoxid bei Raumtemperatur gelöst. Anschließend werden 15,6 g (75,6 mmol) N,N'-Dicyclohexylcarbodiimid zugesetzt und 60 Minuten voraktiviert. Zu der so hergestellten N-Hydroxysuccinimidester-Lösung gibt man eine Lösung von 2,55 g (0,7 mmol) des im Beispiel 12e beschriebenen Tetracosahydrochlorids und 8,5 g Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet.
Ausbeute: 11,8 g (88 % d. Th.)
H₂O-Gehalt (Karl-Fischer): 9,1 %
Gd-Bestimmung (AAS): 19,4 %
MALDI-TOF-Massenspektrum: 17,476 (M+Na⁺)

| Elementaranalyse (berechnet auf wasserfreie Substanz): | | | | |
|---|---|---|---|---|
| ber. | C 40,26 | H 5,35 | N 13,24 | Gd 21,62 |
| gef. | C 40,10 | H 5,51 | N 13,52 | Gd 20,98 |

### Beispiel 26

### a) Gemisch bestehend aus dem Gadoliniumkomplex der 10-(4-Carboxy-1-methyl-2-oxo-3-azabutyl)-1,4,7,10-tetraazacyclododecan-1,4,7-triessigsäure und einem Moläquivalent Natriumbromid

31,74 g (50,4 mmol) des in Beispiel 1f beschriebenen Gd-Komplexes der 10-(4-Carboxy-1-methyl-2-oxo-3-azabutyl)-1,4,7,10-tetraazacyclododecan-1,4,7-triessigsäure werden in 200 ml Wasser gelöst und mit 5,19 g (50,4 mmol) Natriumbromid versetzt.Die klare Lösung wird anschließend gefriergetrocknet und schließlich über Nacht bei 100°C im Vakuum nachgetrocknet. Man erhält ein farbloses Pulver.
Ausbeute: 36,9 g (quantitativ)
H₂O-Gehalt (Karl-Fischer): 3,7 %
Gd-Bestimmung (AAS): 20,9 %

| Elementaranalyse (berechnet auf wasserfreie Substanz): | | | | | | |
|---|---|---|---|---|---|---|
| ber. | C 31,15 | H 4,13 | N 9,56 | Gd 21,46 | Na 3,14 | Br 10,91 |
| gef. | C 30,95 | H 4,20 | N 9,66 | Gd 21,13 | Na 3,31 | Br 10,54 |

### b) Hexatriacontakis-Amid-Konjugat des 36mer-Polyamins aus Beispiel 13d mit dem Gadolinium-Komplex der 10-(4-Carboxy-1-methyl-2-oxo-3-azabutyl)-1,4,7,10-tetraazacyclododecan-1,4,7-triessigsäure

1,84 (0,2 mmol) des im Beispiel 13d beschriebenen 36mer-Benzyloxycarbonylamins werden in Eisessig gelöst und unter Rühren mit 33 prozentigem Bromwasserstoff in Eisessig versetzt. Nach 5 Stunden wird mit Diethylether die begonnene Fällung vervollständigt, das entstandene 36mer-Amin-hydrobromid mit Ether gewaschen, im Vakuum getrocknet und ohne weitere Reinigung in die weiter unten beschriebene Reaktion eingesetzt.
Ausbeute: 1,5 g (quantitativ)

15,8 g (21,6 mmol, dreifacher Überschuß) des im vorstehenden Beispiel 26a beschriebenen Natriumbromid-Mischsalzes mit dem Gd-Komplex der 10-(4-Carboxy-1-methyl-2-oxo-3-azabutyl)-1,4,7,10-tetraazacyclododecan-1,4,7-triessigsäure und 3,73 g (32,4 mmol) N-Hydroxysuccinimid) werden in 100 ml Dimethylsulfoxid gelöst. Anschließend werden 6,7 g (32,4 mmol) N,N'-Dicyclohexylcarbodiimid zugesetzt und 60 Minuten voraktiviert. Zu der so hergestellten N-Hydroxysuccinimidester-Lösung gibt man eine Lösung von 1,5 g (0,2 mmol) des oben beschriebenen Hexatriaconta-hydrobromids und 3,5 g Triethylamin in 10 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON®YM-3 Ultrafiltrationsmembran (cut off 3000 Da) entsalzt und von niedermolekularen Bestandteilen gereinigt. Das Retentat wird anschließend gefriergetrocknet.
Ausbeute: 4,8 g (82 % d. Th.)
H₂O-Gehalt (Karl-Fischer): 9,9 %
Gd-Bestimmung (AAS): 18,6 %
MALDI-TOF-Massenspektrum: 26,428 (M + Na⁺)

| Elementaranalyse (berechnet auf wasserfreie Substanz): | | | | |
|---|---|---|---|---|
| ber. | C 40,53 | H 5,37 | N 13,21 | Gd 21,44 |
| gef. | C 39,46 | H 5,46 | N 12,97 | Gd 21,01 |

### Beispiel 27

### Polyamid-Konjugat von poly-Lysin mit dem Gadoliniumkomplex der 10-(4-Carboxy-1-methyl-2-oxo-3-azabutyl)-1,4,7,10-tetraazacyclododecan-1,4,7-triessigsäure

3,57 g (5 mmol) des im Beispiel 25a beschriebenen Lithium-Mischsalzes mit dem Gd-Komplex der 10-(4-Carboxy-1-methyl-2-oxo-3-azabutyl)-1,4,7,10-tetraazacyclododecan-1,4,7-triessigsäure und 0,58 g (5 mmol) N-Hydroxysuccinimid werden in 25 ml Dimethylsulfoxid gelöst, 1.03 g (5 mmol) N,N'-Dicyclohexylcarbodiimid zugesetzt und 60 Minuten gerührt. Zu der so hergestellten N-Hydroxysuccinimidester-Lösung gibt man eine Lösung von 523 mg (2,5 mmol) poly-Lysin-Hydrobromid (Sigma) und 506 mg (5 mmol) Triethylamin in 5 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen von unlöslichen Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet.
Ausbeute: 1,7 g (82 % d. Th.)
H₂O-Gehalt (Karl-Fischer): 9,9 %
Gd-Bestimmung (AAS): 19,3 %

| Elementaranalyse (berechnet auf wasserfreie Substanz): | | | | |
|---|---|---|---|---|
| ber. | C 40,58 | H 5,45 | N 13,25 | Gd 21,25 |
| gef. | C 40,67 | H 5,25 | N 13,52 | Gd 20,64 |

### Beispiel 28

### a) Gemisch bestehend aus dem Dysprosiumkomplex der 10-(4-Carboxy-1-ethyl-2-oxo-3-azabutyl)-1,4,7,10-tetraazacyclododecan-1,4,7-triessigsäure und einem Moläquivalent Natriumbromid

32,71 g (50,4 mmol) des in Beispiel 5 beschriebenen Dy-Komplexes der 10-(4-Carboxy-1-ethyl-2-oxo-3-azabutyl)-1,4,7,10-tetraazacyclododecan-1,4,7-triessigsäure werden in 200 ml Wasser gelöst und mit 5,19 g (50,4 mmol) Natriumbromid versetzt.Die klare Lösung wird anschließend gefriergetrocknet und schließlich über Nacht bei 100°C im Vakuum nachgetrocknet. Man erhält ein farbloses Pulver.
Ausbeute: 37,9 g (quantitativ)
H₂O-Gehalt (Karl-Fischer): 3,5 %
Dy-Bestimmung (AAS): 20,7 %

| Elementaranalyse (berechnet auf wasserfreie Substanz): | | | | | | |
|---|---|---|---|---|---|---|
| ber. | C 31,95 | H 4,29 | N 9,31 | Dy 21,61 | Na 3,06 | Br 10,63 |
| gef. | C 31,70 | H 4,44 | N 9,08 | Dy 21,09 | Na 3,24 | Br 10,96 |

### b) Dotriacontakis-Amid-Konjugat aus dem 32mer-Dendrimer-amin "DAB(PA)₄(PA)₈(PA)₁₆ (PA)₃₂" und dem Dysprosiumkomplex der 10-(4-Carboxy-1-ethyl-2-oxo-3-azabutyl)-1,4,7,10-tetraazacyclododecan-1,4,7-triessigsäure

3,76 g (5 mmol) des im vorstehenden Beispiel 28a beschriebenen Natriumbromid-Mischsalzes mit dem Dy-Komplex der 10-(4-Carboxy-1-ethyl-2-oxo-3-azabutyl)-1,4,7,10-tetraazacyclododecan-1,4,7-triessigsäure, 696 mg (5 mmol) 4-Nitrophenol und 1,03 g (5 mmol) N,N'-Dicyclohexylcarbodiimid werden in 25 ml Dimethylsulfoxid gelöst und eine Stunde gerührt. Zu der so hergestellten Aktivester-Lösung gibt man 275 mg (0,078 mmol) des in Beispiel VIII der WO 93/14147 beschriebenen 32mer-Dendrimer-Amins und 506 mg (5 mmol) Triethylamin in 5 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen von unlöslichen Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet.
Ausbeute: 1,62 g (83 % d. Th.)
H₂O-Gehalt (Karl-Fischer): 10,0 %
Dy-Bestimmung (AAS): 20,3 %

| Elementaranalyse (berechnet auf wasserfreie Substanz): | | | | |
|---|---|---|---|---|
| ber. | C 40,90 | H 5,76 | N 13,37 | Dy 22,36 |
| gef. | C 40,59 | H 5,98 | N 13,24 | Dy 21,91 |

### Beispiel 29

### Amid-Konjugat von Daunomycin mit dem Gadoliniumkomplex von 1-[1-Methyl-2-oxo-3-aza-4-carboxybutyl]-4,7,10-tris(carboxymethyl)-1,4,7,10-tetraazacyclododecan

1,135 g (1,588 mmol) der Titelverbindung aus Beispiel 25a und 365,8 mg (3,176 mmol) 4-Nitrophenol werden in 10 ml Dimethylsulfoxid gelöst und anschließend auf 10°C abgekühlt. Man gibt bei 10°C 393 mg (1,906 mmol) Dicyclohexylcarbodiimid hinzu und rührt 45 Minuten bei Raumtemperatur. Zu der so hergestellten Suspension gibt man 812,3 mg (1,588 mmol) Daunomycin gelöst in 5 ml Dimethylsulfoxid zu, anschließend 321,4 mg (3,176 mmol) Triethylamin. Man rührt über Nacht bei Raumtemperatur. Zu der erhaltenen Suspension tropft man 200 ml Aceton, filtriert den Niederschlag ab und wäscht ihn 2 mal mit etwas Aceton nach. Der Rückstand wird säulenchromatographisch gereinigt (RP18/Laufmittel: Gradient aus Wasser/Acetonitril/Tetrahydrofuran).
Ausbeute: 1,81 g (87 % d. Th.) farbloses, flockiges Pulver nach Gefriertrocknung Wassergehalt: 6,8 %

| Elementaranalyse (berechnet auf wasserfreie Substanz): | | | | |
|---|---|---|---|---|
| ber. | C 49,19 | H 5,11 | N 7,48 | Gd 14,00 |
| gef. | C 49,01 | H 5,20 | N 7,34 | Gd 13,90 |

### Beispiel 30

### Amid-Konjugat von Adriamycin mit dem Gadoliniumkomplex von 1-[1-Methyl-2-oxo-3-aza-4-carboxybutyl]-4,7,10-tris(carboxymethyl)-1,4,7,10-tetraazacyclododecan

1,135 g (1,588 mmol) der Titelverbindung aus Beispiel 25a und 442 mg (3,176 mmol) 4-Nitrophenol werden in 10 ml Dimethylsulfoxid gelöst und anschließend auf 10°C abgekühlt. Man gibt bei 10°C 393 mg (1,906 mmol) Dicyclohexylcarbodiimid hinzu und rührt 45 Minuten bei Raumtemperatur. Zu der so hergestellten Suspension gibt man 812,3 mg (1,588 mmol) Adriamycin gelöst in 5 ml Dimethylsulfoxid zu, anschließend 321,4 mg (3,176 mmol) Triethylamin. Man rührt über Nacht bei Raumtemperatur. Zu der erhaltenen Suspension tropft man 200 ml Aceton, filtriert den Niederschlag ab und wäscht ihn 2 mal mit etwas Aceton nach. Der Rückstand wird säulenchromatographisch gereinigt (RP18/Laufmittel: Gradient aus Wasser/Acetonitril/Tetrahydrofuran).
Ausbeute: 1,94 g (90 % d. Th.) farbloses, flockiges Pulver nach Gefriertrocknung
Wassergehalt: 9,1 %

| Elementaranalyse (berechnet auf wasserfreie Substanz): | | | | |
|---|---|---|---|---|
| ber. | C 48,50 | H 5,04 | N 7,38 | Gd 13,80 |
| gef. | C 48,35 | H 5,21 | N 7,24 | Gd 13,63 |

### Beispiel 31

### a) Gemisch bestehend aus dem Dysprosiumkomplex der 10-(4-Carboxy-1-methyl-2-oxo-3-azabutyl)-1,4,7,10-tetraazacyclododecan-1,4,7-triessigsäure und einem Moläquivalent Natriumbromid

32,0 g (50,4 mmol) des in Beispiel 2 beschriebenen Dy-Komplexes der 10-(4-Carboxy-1-methyl-2-oxo-3-azabutyl)-1,4,7,10-tetraazacyclododecan-1,4,7-triessigsäure werden in 200 ml Wasser gelöst und mit 5,19 g (50,4 mmol) Natriumbromid versetzt.Die klare Lösung wird anschließend gefriergetrocknet und schließlich über Nacht bei 100°C im Vakuum nachgetrocknet. Man erhält ein farbloses Pulver.
Ausbeute: 37,2 g (quantitativ)
H₂O-Gehalt (Karl-Fischer): 3,5 %
Dy-Bestimmung (AAS): 20,8 %

| Elementaranalyse (berechnet auf wasserfreie Substanz): | | | | | | |
|---|---|---|---|---|---|---|
| ber. | C 30,93 | H 4,10 | N 9,49 | Dy 22,02 | Na 3,12 | Br 10,83 |
| gef. | C 30,81 | H 4,29 | N 9,31 | Dy 21,70 | Na 3,06 | Br 10,42 |

### b) Hexatriacontakis-Amid-Konjugat des 36mer-Polyamins aus Beispiel 13d mit dem Dysprosium-Komplex der 10-(4-Carboxy-1-methyl-2-oxo-3-azabutyl)-1,4,7,10-tetraazacyclododecan-1,4,7-triessigsäure

1,84 (0,2 mmol) des im Beispiel 13d beschriebenen 36mer-Benzyloxycarbonylamins werden in Eisessig gelöst und unter Rühren mit 33 prozentigem Bromwasserstoff in Eisessig versetzt. Nach 5 Stunden wird mit Diethylether die begonnene Fällung vervollständigt, das entstandene 36mer-Amin-hydrobromid mit Ether gewaschen, im Vakuum getrocknet und ohne weitere Reinigung in die weiter unten beschriebene Reaktion eingesetzt.
Ausbeute: 1,5 g (quantitativ)
15,9 g (21,6 mmol, dreifacher Überschuß) des im vorstehenden Beispiel 31a beschriebenen Natriumbromid-Mischsalzes mit dem Dysprosium-Komplex der 10-(4-Carboxy-1-methyl-2-oxo-3-azabutyl)-1,4,7,10-tetraazacyclododeean-1,4,7-triessigsäure, 3,73 g (32,4 mmol) N-Hydroxysuccinimid und 6,7 g (32,4 mmol) N,N'-Dicyclohexylcarbodiimid werden in 100 ml Dimethylsulfoxid bei Raumtemperatur gelöst und 60 Minuten voraktiviert. Zu der so hergestellten N-Hydroxysuccinimidester-Lösung gibt man eine Lösung von 1,5 g (0,2 mmol) des oben beschriebenen Hexatriaconta-hydrobromids und 3,5 g Triethylamin in 10 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON®YM-3 Ultrafiltrationsmembran (cut off 3000 Da) entsalzt und von niedermolekularen Bestandteilen gereinigt. Das Retentat wird anschließend gefriergetrocknet.
Ausbeute: 5,0 g (84 % d. Th.)
H₂O-Gehalt (Karl-Fischer): 10,5 %
Dy-Bestimmung (AAS): 19,6 %
MALDI-TOF-Massenspektrum: 26,616 (M + Na⁺)

| Elementaranalyse (berechnet auf wasserfreie Substanz): | | | | |
|---|---|---|---|---|
| ber. | C 40,24 | H 5,35 | N 13,11 | Dy 22,00 |
| gef. | C 40,02 | H 5,55 | N 13,36 | Dy 21,40 |

### Beispiel 32

### a) Gemisch bestehend aus dem Gadoliniumkomplex der 10-(13-Carboxy-1-methyl-2-oxo-3-azatridecyl)-1,4,7,10-tetraazacyclododecan-1,4,7-triessigsäure und 2 Moläquivalenten Lithiumchlorid

38,1 g (50,4 mmol) des in Beispiel 8c beschriebenen Gd-Komplexes der 10-(13-Carboxy-1-methyl-2-oxo-3-azatridecyl)-1,4,7,10-tetraazacyclododecan-1,4,7-triessigsäure werden in 250 ml Wasser gelöst und mit 4,27 g (100,8 mmol) Lithiumchlorid versetzt.Die klare Lösung wird anschließend gefriergetrocknet und schließlich über Nacht bei 100°C im Vakuum nachgetrocknet. Man erhält ein farbloses Pulver.
Ausbeute: 42,4 g (quantitativ)
H₂O-Gehalt (Karl-Fischer): 3,5 %
Gd-Bestimmung (AAS): 18,0 %

| Elementaranalyse (berechnet auf wasserfreie Substanz): | | | | | | |
|---|---|---|---|---|---|---|
| ber. | C 40,00 | H 5,75 | N 8,33 | Gd 18,70 | Li 1,65 | Cl 8,43 |
| gef. | C 39,73 | H 5,99 | N 8,51 | Gd 18,17 | Li 1,58 | Cl 8,66 |

### b) Hexaamid-Konjugat von 6,6',6'',6''',6'''',6'''''-Hexaamino-6,6',6'',6''',6'''',6''''''-hexadeoxy-α-cyclodextrin mit dem Gadoliniumkomplex der 10-(13-Carboxy-1-methyl-2-oxo-3-azatridecyl)-1,4,7,10-tetraazacyclododecan-1,4,7-triessigsäure

4,2 g (5 mmol) des im vorstehenden Beispiel 32a beschriebenen Lithium-Mischsalzes mit dem Gadoliniumkomplex der 10-(5-Carboxy-1-methyl-2-oxo-3-azatridecyl)-1,4,7,10-tetraazacyclododecan-1,4,7-triessigsäure, 1,15g (10 mmol) N-Hydroxysuccinimid und 1,24 g (6 mmol) Dicyclohexylcarbodiimid werden in 30 ml Dimethylsulfoxid gelöst und 8 Stunden bei Raumtemperatur gerührt. Die so hergestellte N-Hydroxysuccinimidesterlösung wird zu einer Suspension von 315 mg (0,25 mmol) 6,6',6'',6''',6'''',6'''''-Hexaamino-6,6',6'',6''',6'''',6'''''hexadeoxy-α-cyclodextrin [J.Boger,R.J.Corcoran und J.-M.Lehn, Helv. Chim. Acta 61, 2190 - 2218 (1978)] in Dimethylsulfoxid zugegeben und über Nacht bei Raumtemperatur gerührt. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet. Man erhält ein farbloses Pulver.
Ausbeute: 1,35 g (90 % d. Th.)
H₂O-Gehalt (Karl-Fischer): 9,5 %
Gd-Bestimmung (AAS): 16,6 %

| Elementaranalyse (berechnet auf wasserfreie Substanz): | | | | |
|---|---|---|---|---|
| ber. | C 45,42 | H 6,39 | N 9,35 | Gd 17,49 |
| gef. | C 45,13 | H 6,20 | N 9,57 | Gd 17,11 |

### Beispiel 33

### Amid-Konjugat von 3'-Amino-3'-deoxyguanosin mit dem Gadoliniumkomplex von 1-[1-Methyl-2-oxo-3-aza-4-carboxybutyl]-4,7,10-tris(carboxymethyl)-1,4,7,10-tetraazacyclododecan

1,163 g (1,588 mmol) der Titelverbindung aus Beispiel 26a und 365,8 mg (3,176 mmol) N-Hydroxysuccinimid werden in 10 ml Dimethylsulfoxid gelöst und anschließend auf 10°C abgekühlt. Man gibt bei 10°C 393 mg (1,906 mmol) Dicyclohexylcarbodiimid hinzu und rührt 45 Minuten bei Raumtemperatur. Zu der so hergestellten N-Hydroxysuccinimidester-Lösung gibt man 422,8 mg (1,588 mmol) 3'-Amino-3'-deoxyguanosin, gelöst in 5 ml Dimethylsulfoxid zu, anschließend 321,4 mg (3,176 mmol) Triethylamin. Man rührt über Nacht bei Raumtemperatur. Zu dieser Lösung tropft man 200 ml Aceton, Filtriert den Niederschlag ab und wäscht ihn 2 mal mit etwas Aceton nach. Der Rückstand wird säulenchromatographisch gereinigt (RP18/Laufmittel: Gradient aus Wasser/Acetonitril/Tetrahydrofuran).
Ausbeute: 1,36 g (90 % d. Th.) farbloses, flockiges Pulver nach Gefriertrocknung Wassergehalt: 7,4 %

| Elementaranalyse (berechnet auf wasserfreie Substanz): | | | | |
|---|---|---|---|---|
| ber. | C 39,67 | H 4,82 | N 17,55 | Gd 17,91 |
| gef. | C 39,48 | H 4,94 | N 17,38 | Gd 17,76 |

### Beispiel 34

### Amid-Konjugat von Dehydroabiethylamin mit dem Gadoliniumkomplex von 1-[1-Methyl-2-oxo-3-aza-4-carboxybutyl]-4,7,10-tris(carboxymethyl)-1,4,7,10-tetraazacyclododecan

1,163 g (1,588 mmol) der Titelverbindung aus Beispiel 26a und 365,8 mg (3,176 mmol) N-Hydroxysuccinimid werden in 10 ml Dimethylsulfoxid gelöst und anschließend auf 10°C abgekühlt. Man gibt bei 10°C 393 mg (1,906 mmol) Dicyclohexylcarbodiimid hinzu und rührt 45 Minuten bei Raumtemperatur. Zu der so hergestellten Suspension gibt man 422,8 mg (1,588 mmol) Dehydroabiethylamin, gelöst in 5 mi Dimethylsulfoxid zu, anschließend 321,4 mg (3,176 mmol) Triethylamin. Man rührt über Nacht bei Raumtemperatur. Zu dieser Suspension tropft man 200 ml Aceton, filtriert den Niederschlag ab und wäscht ihn 2 mal mit etwas Aceton nach. Der Rückstand wird säulenchromatographisch gereinigt (RP18/Laufmittel:
Gradient aus Wasser/ Acetonitril/Tetrahydrofuran).
Ausbeute: 1,40 g (92 % d. Th.) glasiger Feststoff nach Gefriertrocknung
Wassergehalt: 6,5 %

| Elementaranalyse (berechnet auf wasserfreie Substanz): | | | | |
|---|---|---|---|---|
| ber. | C 52,21 | H 6,63 | N 9,37 | Gd 17,53 |
| gef. | C 52,35 | H 6,81 | N 9,20 | Gd 17,38 |

### Beispiel 35

### Amid-Konjugat von Ampicillin mit dem Gadoliniumkomplex von 1-[1-Methyl-2-oxo-3-aza-4-carboxybutyl]-4,7,10-tris(carboxymethyl)-1,4,7,10-tetraazacyclododecan

1,135 g (1,588 mmol) der Titelverbindung aus Beispiel 25a und 365,8 mg (3,176 mmol) N-Hydroxysuccinimid werden in 10 ml Dimethylsulfoxid gelöst und anschließend auf 10°C abgekühlt. Man gibt bei 10°C 393 mg (1,906 mmol) Dicyclohexylcarbodiimid hinzu und rührt 45 Minuten bei Raumtemperatur. Zu der so hergestellten Suspension gibt man 554,9 mg (1,588 mmol) Ampicillin, gelöst in 5 ml Dimethylsulfoxid zu, anschließend 482 mg (4,764 mmol) Triethylamin. Man rührt über Nacht bei Raumtemperatur. Zu dieser Suspension tropft man 200 ml Aceton, filtriert den Niederschlag ab und wäscht ihn 2 mal mit etwas Aceton nach. Der Rückstand wird säulenchromatographisch gereinigt (RP18/Laufmittel: Gradient aus Wasser/ Acetonitril/Tetrahydrofuran /+ 0,5 % Trifluoressigsäure).
Ausbeute: 1,54 g (90 % d. Th.) farbloses, flockiges Pulver nach Gefriertrocknung
Wassergehalt: 10,0 %

| Elementaranalyse (berechnet auf wasserfreie Substanz): | | | | | |
|---|---|---|---|---|---|
| ber. | C 45,76 | H 4,88 | N 10,10 | S 3,30 | Gd 16,19 |
| gef. | C 45,61 | H 5,10 | N 10,00 | S 3,20 | Gd 16,05 |

### Beispiel 36

### Amid-Konjugat von Pentapeptid NH₂-Val-Leu-Phe-Phe-Ala-OH mit dem Gadoliniumkomplex von 1-[1-Methyl-2-oxo-3-aza-4-carboxybutyl]-4,7,10-tris(carboxymethyl)-1,4,7,10-tetraazacyclododecan

1,135 g (1,588 mmol) der Titelverbindung aus Beispiel 25a und 365,8 mg (3,176 mmol) N-Hydroxysuccinimid werden in 10 ml Dimethylsulfoxid gelöst und anschließend auf 10°C abgekühlt. Man gibt bei 10°C 393 mg (1,906 mmol) Dicyclohexylcarbodiimid hinzu und rührt 45 Minuten bei Raumtemperatur. Zu der so hergestellten Suspension gibt man 946 mg (1,588 mmol) des Pentapeptids NH₂-Val-Leu-Phe-Phe-Ala-OH (hergestellt nach der Festphasentechnik), gelöst in 5 ml Dimethylsulfoxid zu, anschließend 321,4 mg (3,176 mmol) Triethylamin. Man rührt über Nacht bei Raumtemperatur. Zu dieser Suspension tropft man 200 ml Aceton, filtriert den Niederschlag ab und wäscht ihn 2 mal mit etwas Aceton nach. Der Rückstand wird säulenchromatographisch gereinigt (RP18/Laufmittel: Gradient aus Wasser/Acetonitril/Tetrahydrofuran).
Ausbeute: 1,81 g (87 % d. Th.) farbloses, flockiges Pulver nach Gefriertrocknung Wassergehalt: 8,1 %

| Elementaranalyse (berechnet auf wasserfreie Substanz): | | | | |
|---|---|---|---|---|
| ber. | C 50,73 | H 6,09 | N 11,60 | Gd 13.02 |
| gef. | C 50,54 | H 6,19 | N 11,41 | Gd 12,80 |

### Beispiel 37

### Amid-Konjugat von N-Methylglucosamin mit dem Gadoliniumkomplex von 1-[1-Methyl-2-oxo-3-aza-4-carboxybutyl]-4,7,10-tris(carboxymethyl)-1,4,7,10-tetraazacyclododecan

1,163 g (1,588 mmol) der Titelverbindung aus Beispiel 26a werden in 10 ml Dimethylsulfoxid gelöst und anschließend auf 10°C abgekühlt. Man gibt bei 10°C 310 mg (1,588 mmol) D(-)-N-Methylglucosamin und 785,4 mg (3,176 mmol) 2-Ethoxy-1-ethoxycarbonyl-1,2-dihydrochinolin (EEDQ) zu. Man rührt über Nacht bei Raumtemperatur. Zu dieser Lösung tropft man 200 ml Aceton, filtriert den Niederschlag ab und wäscht ihn 2 mal mit etwas Aceton nach. Der Rückstand wird säulenchromatographisch gereinigt (RP18/Laufmittel: Gradient aus Wasser/Acetonitril/Tetrahydrofuran).
Ausbeute: 1,30 g (93 % d. Th.) farbloses, flockiges Pulver nach Gefriertrocknung Wassergehalt: 8,5 %

| Elementaranalyse (berechnet auf wasserfreie Substanz): | | | | |
|---|---|---|---|---|
| ber. | C 38,70 | H 5,62 | N 10,41 | Gd 19,49 |
| gef. | C 38,55 | H 5,80 | N 10,30 | Gd 19,29 |

## Patentansprüche

1. Verfahren zur Herstellung von Metallkomplexcarbonsäureamiden enthaltend das 1, 4, 7, 10-Tetraazacyclododekan-Gerüst, **dadurch gekennzeichnet, daß** ein Metallkomplexcarbonsäure-Gemisch bestehend aus der Metallkomplexcarbonsäure
- wobei das Metall aus der Gruppe der Lanthaniden, Eisen, Mangan, Yttrium und Wismut ausgewählt wird - und mindestens einem lösungsvermittelnden Stoff in Dimethylsulfoxid (DMSO) mit einem wasserabspaltenden Reagenz gegebenenfalls unter Zusatz eines Kupplungs-Hilfsstoffs vorbehandelt wird und anschließend mit einem Amin der allgemeinen Formel I
A(NH₂)ₙ (I),
worin
A für den Rest eines natürlichen oder synthetischen Amins, wobei das Amin aus der Gruppe der Antibiotika, Nukleoside, Aminoterpene, Aminoporphyrine, Aminosteroide, Aminozucker, Dendrimere, Proteine, Polylysin, Aminopolysaccharide, Polyvinylamine, Polyalkylamine, Poly[N(2-aminoethyl)]methacrylamide, Polynucleotide und Polypeptide ausgewählt wird und
n für die Zahlen 1 bis 100 stehen,
umgesetzt wird, wobei die Reaktion in Lösung bei Temperaturen von 0 bis 70°C stattfindet.

2. Verfahren gemäß Anspruch 1, **dadurch gekennzeichnet, daß** das Metallkomplexcarbonsäure-Gemisch in situ durch Zusatz von lösungsvermittelndem/n Stoff(en) zur DMSO-Suspension von Metallkomplexcarbonsäure, wasserabspaltendem Reagenz und gegebenenfalls einem Kupplungs-Hilfsstoff gebildet wird.

3. Verfahren gemäß Anspruch 1, **dadurch gekennzeichnet, daß** zunächst das Metallkomplexcarbonsäure-Gemisch hergestellt und isoliert wird und dieses dann unter Zusatz eines wasserabspaltenden Reagenzes und gegebenenfalls eines Kupplungs-Hilfsstoffes in DMSO vorbehandelt wird.

4. Verfahren gemäß Anspruch 1, **dadurch gekennzeichnet, daß** das Amin ohne Lösungsmittel zur DMSO-Lösung des vorbehandelten Metallkomplexcarbonsäure-Gemisches gegeben wird.

5. Verfahren gemäß Anspruch 1, **dadurch gekennzeichnet, daß** das Amin in gelöster Form zur DMSO-Lösung des vorbehandelten Metallkomplexcarbonsäure-Gemisches gegeben wird.

6. Verfahren gemäß Anspruch 5, **dadurch gekennzeichnet, daß** das Amin gelöst in DMSO, Wasser oder in mit Wasser gemischten Lösungsmitteln ist.

7. Verfahren gemäß Anspruch 1, **dadurch gekennzeichnet, daß** die Vorbehandlung mit dem wasserabspaltenden Reagenz bei Temperaturen von 0 bis 50° C und Reaktionszeiten zwischen 1 und 24 Stunden erfolgt.

8. Verfahren gemäß Anspruch 7, **dadurch gekennzeichnet, daß** die Vorbehandlung bei Raumtemperatur und Reaktionszeiten zwischen 3 und 12 Stunden erfolgt.

9. Verfahren gemäß Anspruch 1, **dadurch gekennzeichnet, daß** die Umsetzung mit dem Amin der allgemeinen Formel I bei Temperaturen von 0 bis 70° C und Reaktionszeiten zwischen 1 und 48 Stunden erfolgt.

10. Verfahren gemäß Anspruch 9, **dadurch gekennzeichnet, daß** die Umsetzung bei Temperaturen von 30 bis 60° C und Reaktionszeiten zwischen 8 und 24 Stunden erfolgt.

11. Verfahren gemäß Anspruch 1, **dadurch gekennzeichnet, daß** die lösungsvermittelnden Stoffe ausgewählt werden aus Alkali-, Erdalkali-, Trialkylammonium-, Tetraalkylammoniumsalzen, Harnstoffen, N-Hydroxyimiden, Hydroxyaryltriazolen, substituierten Phenolen und Salzen von heterocyclischen Aminen.

12. Verfahren gemäß Anspruch 1, **dadurch gekennzeichnet, daß** n für die Zahl 1 steht.

13. Verfahren gemäß Anspruch 1, **dadurch gekennzeichnet, daß** n für die Zahlen 2 bis 50 steht.

14. Verfahren gemäß Anspruch 1, **dadurch gekennzeichnet, daß** n für die Zahlen 12 bis 36 steht.

15. Verfahren gemäß Anspruch 11, **dadurch gekennzeichnet, daß** die lösungsvermittelnden Stoffe ausgewählt werden aus den Verbindungen Lithiumchlorid, Lithiumbromid, Lithiumiodid, Natriumbromid und Natriumiodid.

16. Verfahren gemäß Anspruch 1, **dadurch gekennzeichnet, daß** die Metallkomplexcarbonsäure für einen Gadolinium- oder Dysprosium-Komplex steht.

17. Verfahren gemäß Anspruch 1, **dadurch gekennzeichnet, daß** die Metallkomplexcarbonsäure für eine Verbindung der allgemeinen Formel II 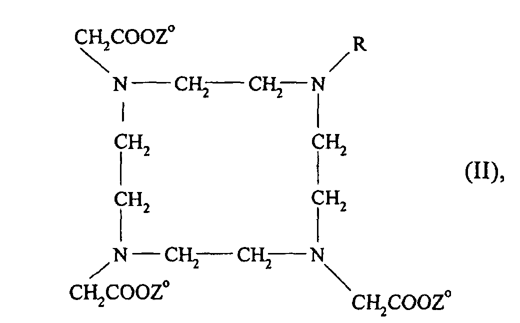 wobei
Z⁰ für ein Metallionenäquivalent der Ordnungszahlen 25, 26, 39, 57-71, 83 und
R für eine CHX¹-CO-NH-CHY¹-(CH₂)_{f}-COOH-Gruppe steht, worin X¹ und Y¹ unabhängig voneinander ein Wasserstoffatom, einen geradkettigen oder verzweigten C₁-C₇-Alkylrest, eine Phenyl- oder Benzylgruppe und
f die Ziffern 0 bis 9 bedeuten, steht.

18. Verfahren gemäß Anspruch 17, **dadurch gekennzeichnet, daß** die Metallkomplexcarbonsäure der allgemeinen Formel II für einen Gadolinium- oder Dysprosium-Komplex der 10-[4-Carboxy-1-methyl-2-oxo-3-azabutyl]-1,4,7,10-tetraazacyclododecan-1,4,7-triessigsäure steht.

19. Verfahren gemäß Anspruch 1, **dadurch gekennzeichnet, daß** der Rest A des Amins der allgemeinen Formel I keine protonierbaren Aminogruppen enthält.

20. Verfahren gemäß Anspruch 19, **dadurch gekennzeichnet, daß** das Amin der allgemeinen Formel I für ein 24mer Polyamin auf der Basis des N,N,N',N',N",N"-Hexakis[2-(trilysyl-amino)-ethyl]-trimesinsäuretriamids steht.

21. Verfahren gemäß Anspruch 1, **dadurch gekennzeichnet, daß** das Metallkomplexcarbonsäure-Gemisch bis zu 5 Moläquivalente des lösungsvermittelnden Stoffes bezogen auf die Metallkomplexcarbonsäure enthält.

22. Verfahren gemäß Anspruch 1, **dadurch gekennzeichnet, daß** das Metallkomplexcarbonsäure-Gemisch 0,5 bis 2 Moläquivalente des lösungsvermittelnden Stoffes bezogen auf die Metallkomplexcarbonsäure enthält.

23. Verfahren gemäß Anspruch 1, **dadurch gekennzeichnet, daß** man als wasserabspaltendesReagenz Carbodiimide oder Onium-Reagenzien verwendet.

24. Verfahren gemäß Anspruch 23, **dadurch gekennzeichnet, daß** man als Carbodiimid Dicyclohexylcarbodiimid verwendet.

25. Verfahren gemäß Anspruch 1, **dadurch gekennzeichnet, daß** man als Kupplungs-Hilfsstoff 4-Nitrophenol, N-Hydroxysuccinimid, 1-Hydroxybenzotriazol, 1-Hydroxy-7-azabenzotriazol, 3,5-Dinitrophenol oder Pentafluorphenol verwendet.

26. Verfahren gemäß Anspruch 25, **dadurch gekennzeichnet, daß** man als Kupplungs-Hilfsstoff 4-Nitrophenol verwendet.

27. Verfahren gemäß Anspruch 1, **dadurch gekennzeichnet, daß** zunächst ein Gemisch aus dem Gadolinium-Komplex der 10-[4-Carboxy-1-methyl-2-oxo-3-azabutyl)-1,4,7,10-tetraazacyclododecan-1,4,7-trieessigsäure und 1 bis 2 Äquivalent(en) Natriumbromid hergestellt und isoliert wird und dieses dann unter Zusatz von 4-Nitrophenol und Dicyclohexylcarbodiimid in DMSO bei Raumtemperatur und Reaktionszeiten zwischen 3 und 12 Stunden vorbehandelt wird und anschließend gegebenenfalls unter Wasserzusatz mit dem 24mer Polyamin auf der Basis des N,N,N',N',N",N"-Hexakis[2-(trilysyl-amino)-ethyl]-trimesinsäuretriamids bei 20 bis 60° C und Reaktionszeiten zwischen 8 und 24 Stunden umgesetzt wird.

28. Verfahren gemäß Anspruch 1, **dadurch gekennzeichnet, daß** zunächst ein Gemisch aus dem Gadolinium-Komplex der 10-[4-Carboxy-1-methyl-2-oxo-3-azabutyl)-1,4,7,10-tetraazacyclododecan-1,4,7-trieessigsäure und 1 bis 2 Äquivalent(en) Lithiumchlorid hergestellt und isoliert wird und dieses dann unter Zusatz von 4-Nitrophenol und Dicyclohexylcarbodiimid in DMSO bei Raumtemperatur und Reaktionszeiten zwischen 3 und 12 Stunden vorbehandelt wird und anschließend gegebenenfalls unter Wasserzusatz mit dem 24mer Polyamin auf der Basis des N,N,N',N',N",N"-Hexakis[2-(trilysyl-amino)-ethyl]-trimesinsäuretriamids bei 20 bis 60° C und Reaktionszeiten zwischen 8 und 24 Stunden umgesetzt wird.

## Claims

1. Process for the production of metal-complex carboxylic acid amides containing the 1,4,7,10-tetraazacyclododecane skeleton, **characterized in that** a metal-complex carboxylic acid mixture that consists of the metal-complex carboxylic acid (where the metal is selected from the group consisting of lanthanides, iron, manganese, yttrium and bismuth) and at least one solubilizing substance is, pretreated in dimethyl sulfoxide (DMSO) with a dehydrating reagent, optionally in the presence of a coupling assistant, and is then reacted with an amine of the general formula I
A(NH₂)ₙ (I),
in which
A stands for the radical of a natural or synthetic amine, the amine being selected from the group consisting of antibiotics, nucleosides, amino terpenes, amino porphyrins, amino steroids, amino sugars, dendrimers, proteins, polylysine, amino polysaccharides, polyvinylamines, polyalkylamines, poly-[N(2-aminoethyl)]methacrylamides, polynucleotides and polypeptides and n stands for numbers 1 to 100,
the reaction taking place in solution at temperatures of 0 to 70°C.

2. Process according to Claim 1, **characterized in that** the metal-complex carboxylic acid mixture is formed in situ by addition of one or more solubilizing substance(s) to the DMSO suspension of metal-complex carboxylic acid, dehydrating reagent, and optionally a coupling assistant.

3. Process according to Claim 1, **characterized in that** initially the metal-complex carboxylic acid mixture is produced and isolated and then pretreated by addition of a dehydrating reagent and optionally of a coupling assistant in DMSO.

4. Process according to Claim 1, **characterized in that** the amine is added without solvent to the DMSO solution of the pretreated metal-complex carboxylic acid mixture.

5. Process according to Claim 1, **characterized in that** the amine is added in dissolved form to the DMSO solution of the pretreated metal-complex carboxylic acid mixture.

6. Process according to Claim 5, **characterized in that** the amine has been dissolved in DMSO, water or in solvents which have been mixed with water.

7. Process according to Claim 1, **characterized in that** the pretreatment with the dehydrating reagent is carried out at 0 to 50°C and reaction times between 1 and 24 hours.

8. Process according to Claim 7, **characterized in that** the pretreatment is carried out at room temperature and reaction times between 3 and 12 hours.

9. Process according to Claim 1, **characterized in that** the reaction with the amine of the general formula I is carried out at temperatures of 0 to 70°C and reaction times between 1 and 48 hours.

10. Process according to Claim 9, **characterized in that** the reaction is carried out at temperatures of 30 to 60°C and reaction times between 8 and 24 hours.

11. Process according to Claim 1, **characterized in that** the solubilizing substances are selected from alkali metal salts, alkaline earth metal salts, trialkylammonium salts, tetraalkylammonium salts, ureas, N-hydroxyimides, hydroxyaryltriazoles, substituted phenols and salts of heterocyclic amines.

12. Process according to Claim 1, **characterized in that** n stands for the number 1.

13. Process according to Claim 1, **characterized in that** n stands for the numbers 2 to 50.

14. Process according to Claim 1, **characterized in that** n stands for the numbers 12 to 36.

15. Process according to Claim 11, **characterized in that** the solubilizing substances are selected from the compounds lithium chloride, lithium bromide, lithium iodide, sodium bromide and sodium iodide.

16. Process according to Claim 1, **characterized in that** the metal-complex carboxylic acid stands for a gadolinium or dysprosium complex.

17. Process according to Claim 1, **characterized in that** the metal-complex carboxylic acid stands for a compound of the general formula II 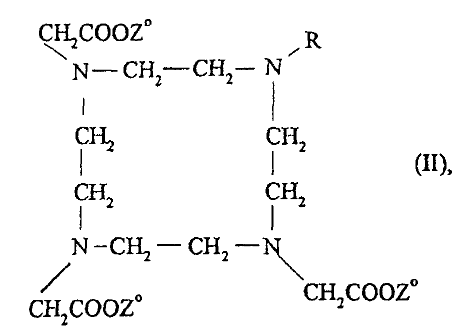 where
Z⁰ stands for a metal ion equivalent of atomic numbers 25, 26, 39, 57-71, 83 and
R stands for a CHX¹-CO-NH-CHY¹-(CH₂)_{f}-COOH group, in which X¹ and Y¹, independently of one another, mean a hydrogen atom, a straight-chain or branched C₁-C₇ alkyl radical, a phenyl or benzyl group, and f means numbers 0 to 9.

18. Process according to Claim 17, **characterized in that** the metal-complex carboxylic acid of the general formula II stands for a gadolinium or dysprosium complex of 10-[4-carboxy-1-methyl-2-oxo-3-azabutyl]-1,4,7,10-tetraazacyclodecane-1,4,7-triacetic acid.

19. Process according to Claim 1, **characterized in that** the radical A of the amine of the general formula I contains no protonatable amino groups.

20. Process according to Claim 19, **characterized in that** the amine of the general formula I stands for a 24-mer polyamine based on N,N,N',N',N",N"-hexakis[2-(trilysyl-amino)-ethyl]trimesic acid triamide.

21. Process according to Claim 1, **characterized in that** the metal-complex carboxylic acid mixture contains up to 5 mol equivalents of the solubilizing substance relative to the metal-complex carboxylic acid.

22. Process according to Claim 1, **characterized in that** the metal-complex carboxylic acid mixture contains from 0.5 to 2 mol equivalents of the solubilizing substance relative to the metal-complex carboxylic acid.

23. Process according to Claim 1, **characterized in that** carbodiimides or onium reagents are used as dehydrating reagent.

24. Process according to Claim,23, **characterized in that** dicyclohexylcarbodiimide is used as carbodiimide.

25. Process according to Claim 1, **characterized in that** 4-nitrophenol, N-hydroxysuccinimide, 1-hydroxybenzotriazole, 1-hydroxy-7-azabenzotriazole, 3,5-dinitrophenol or pentafluorophenol is used as coupling assistant.

26. Process according to Claim 25, **characterized in that** 4-nitrophenol is used as coupling assistant.

27. Process according to Claim 1, **characterized in that** initially a mixture of the gadolinium complex of 10-[4-carboxy-1-methyl-2-oxo-3-azabutyl]-1,4,7,10-tetraazacyclododecane-1,4,7-triacetic acid and 1 to 2 equivalents of sodium bromide is produced and isolated and then pretreated by addition of 4-nitrophenol and dicyclohexylcarbodiimide in DMSO at room temperature and reaction times between 3 and 12 hours and subsequently reacted, in the presence or absence of added water, with the 24-mer polyamine based on N,N,N',N',N",N"-hexakis[2-(trilysyl-amino)-ethyl]-trimesic acid triamide at 20 to 60°C and reaction times between 8 and 24 hours.

28. Process according to Claim 1, **characterized in that** initially a mixture of the gadolinium complex of 10-[4-carboxy-1-methyl-2-oxo-3-azabutyl]-1,4,7,10-tetraazacyclododecane-1,4,7-triacetic acid and 1 to 2 equivalents of lithium chloride is produced and isolated and then pretreated by addition of 4-nitrophenol and dicyclohexylcarbodiimide in DMSO at room temperature and reaction times between 3 and 12 hours and subsequently reacted, in the presence or absence of added water, with the 24-mer polyamine based on N,N,N',N',N",N"-hexakis[2-(trilysyl-amino)-ethyl]-trimesic acid triamide at 20 to 60°C and reaction times between 8 and 24 hours.

## Revendications

1. Procédé de préparation des amides d'acide carboxylique à complexe de métal contenant la structure de 1,4,7,10-tétra-azacyclododécane, **caractérisé en ce qu'**un mélange d'acide carboxylique à complexe de métal constitué d'acide carboxylique à complexe de métal - dans lequel le métal est sélectionné parmi le groupe des lanthanides, du fer, du manganèse, de l'yttrium et du bismuth - et d'au moins d'une matière solubilisante dans du diméthylsulfoxyde (DMSO) est traité au préalable avec un réactif déshydratant éventuellement avec addition d'un composé auxiliaire d'accouplement, puis mis à réagir avec une amine de la formule générale I :
A(NH₂)ₙ (I)
dans laquelle
"A" représente le reste d'une amine naturelle ou synthétique, dans lequel l'amine est sélectionnée parmi le groupe des antibiotiques, des nucléosides, des amino-terpènes, des amino-porphyrines, des amino-stéroïdes, des sucres aminés, des dendrimères, des protéines, de la polylysine, des aminopolysaccharides, des polyvinylamines, des polyalkylamines, des poly-[N(2-amino-éthyl)]-méthacrylamides, des polynucléotides et des polypeptides et
"n" représente un nombre entre 1 et 100,
la réaction ayant lieu dans une solution à des températures entre 0 et 70°C.

2. Procédé selon la revendication 1, **caractérisé en ce que** le mélange d'acide carboxylique à complexe de métal in situ est formé par l'addition d'une/des matière(s) solubilisante(s) à la suspension de DMSO d'acide carboxylique à complexe de métal, d'un réactif déshydratant, et éventuellement d'un composé auxiliaire d'accouplement.

3. Procédé selon la revendication 1, **caractérisé en ce que** le mélange d'acide carboxylique à complexe de métal est d'abord préparé et isolé, et **en ce que** celui-ci est ensuite traité au préalable dans du DMSO avec addition d'un réactif déshydratant, et éventuellement d'un composé auxiliaire d'accouplement.

4. Procédé selon la revendication 1, **caractérisé en ce que** l'amine est ajoutée sans solvant à la solution de DMSO du mélange d'acide carboxylique à complexe de métal traité au préalable.

5. Procédé selon la revendication 1, **caractérisé en ce que** l'amine est ajoutée sous forme dissoute dans la solution de DMSO du mélange d'acide carboxylique à complexe de métal traité au préalable.

6. Procédé selon la revendication 5, **caractérisé en ce que** l'amine est dissoute dans du DMSO, de l'eau ou dans des solvants mélangés avec de l'eau.

7. Procédé selon la revendication 1, **caractérisé en ce que** le traitement préalable avec le réactif solubilisant s'effectue à des températures entre 0 et 50°C et avec des temps de réaction entre 1 et 24 heures.

8. Procédé selon la revendication 7, **caractérisé en ce que** le traitement préalable s'effectue à la température ambiante et avec des temps de réaction entre 3 et 12 heures.

9. Procédé selon la revendication 1, **caractérisé en ce que** la transformation avec l'amine de la formule générale I s'effectue à des températures entre 0 et 70°C et avec des temps de réaction entre 1 et 48 heures.

10. Procédé selon la revendication 9, **caractérisé en ce que** la transformation s'effectue à des températures entre 30 et 60°C et avec des temps de réaction entre 8 et 24 heures.

11. Procédé selon la revendication 1, **caractérisé en ce que** les matières solubilisantes sont choisies parmi les sels alcalins, alcalinoterreux, de trialkylammonium, de tétraalkylammonium, les urées, les N-hydroxyimides, les hydroxyaryltriazoles, les phénols substitués et les sels d'amines hétérocycliques.

12. Procédé selon la revendication 1, **caractérisé en ce que** "n" correspond au nombre 1.

13. Procédé selon la revendication 1, **caractérisé en ce que** "n" correspond aux nombres de 2 à 50.

14. Procédé selon la revendication 1, **caractérisé en ce que** "n" correspond aux nombres de 12 à 36.

15. Procédé selon la revendication 11, **caractérisé en ce que** les matières solubilisantes sont choisies parmi les composés chlorure de lithium, bromure de lithium, iodure de lithium, bromure de sodium et iodure de sodium.

16. Procédé selon la revendication 1, **caractérisé en ce que** l'acide carboxylique à complexe de métal représente le complexe de gadolinium ou de dysprosium.

17. Procédé selon la revendication 1, **caractérisé en ce que** l'acide carboxylique à complexe de métal représente un composé de formule générale II 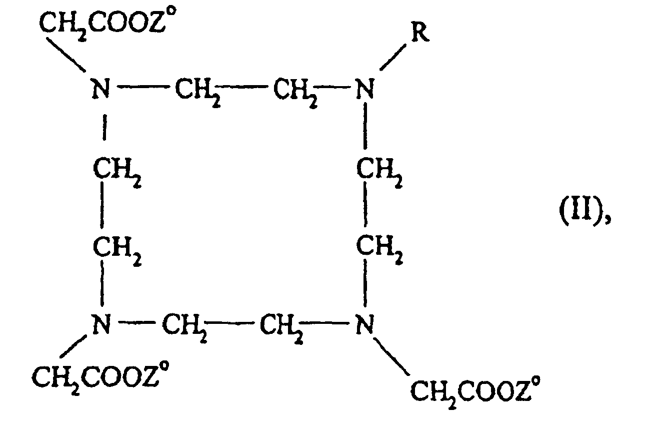 dans laquelle
"Z°" représente un équivalent d'ion métallique de nombres atomiques 25, 26, 39, 57-71, 83 et
"R" représente un groupe CHX¹-CO-NH-CHY¹-(CH₂)_{f}-COOH, où
"X¹" et "Y¹" indépendamment l'un de l'autre signifient un atome d'hydrogène, un résidu alkyle en C₁-C₇ linéaire ou ramifié, un groupe phényle ou benzyle, et
"f" signifie les chiffres de 0 à 9.

18. Procédé selon la revendication 17, **caractérisé en ce que** l'acide carboxylique à complexe de métal de la formule générale II représente un complexe de dysprosium ou gadolinium l'acide 10-[4-carboxy-1-méthyl-2-oxo-3-azabutyl]-1,4,7-triacétique.

19. Procédé selon la revendication 1, **caractérisé en ce que** le reste "A" de l'amine de la formule générale I ne contient aucun groupe aminé capable de recevoir un proton.

20. Procédé selon la revendication 19, **caractérisé en ce que** l'amine de la formule générale I représente une polyamine 24-mère à base de triamide d'acide N,N,N',N',N",N"-hexakis[2-(trilysylamino)-éthyl]-trimésique.

21. Procédé selon la revendication 1, **caractérisé en ce que** le mélange d'acide carboxylique à complexe de métal contient jusqu'à 5 équivalents molaires de la matière solubilisante par rapport à l'acide carboxylique à complexe de métal.

22. Procédé selon la revendication 1, **caractérisé en ce que** le mélange d'acide carboxylique à complexe de métal contient de 0,5 à 2 équivalents molaires de la matière solubilisante par rapport à l'acide carboxylique à complexe de métal.

23. Procédé selon la revendication 1, **caractérisé en ce qu'**on utilise des carbodiimides ou des réactifs onium en tant que réactifs déshydratants.

24. Procédé selon la revendication 23, **caractérisé en ce qu'**on utilise du dicyclohexylcarbodiimide en tant que carbodiimide.

25. Procédé selon la revendication 1, **caractérisé en ce qu'**on utilise comme composé auxiliaire d'accouplement du 4-nitrophénol, du N-hydroxysuccinimide, du 1-hydroxybenzotriazole, du 1-hydroxy-7-azabenzotriazole, du 3,5-dinitrophénol ou du pentafluorophénol.

26. Procédé selon la revendication 25, **caractérisé en ce qu'**on utilise comme composé auxiliaire d'accouplement du 4-nitrophénol.

27. Procédé selon la revendication 1, **caractérisé en ce qu'**un mélange du complexe de gadolinium de l'acide 10-[4-carboxy-1-méthyl-2-oxo-3-azabutyl]-1,4,7,10-tétra-azacyclododécane-1,4,7-triacétique et de 1 à 2 équivalent(s) de bromure de sodium est d'abord préparé et isolé et que celui-ci est ensuite traité au préalable par l'addition de 4-nitrophénol et de dicyclohexylcarbodiimide dans du DMSO à température ambiante et avec des temps de réaction entre 3 et 12 heures, et est ensuite mis à réagir éventuellement avec addition d'eau avec la polyamine 24-mère à base de triamide d'acide N,N,N',N',N",N"-hexakis[2-(trilysylamino)-éthyl]-trimésique entre 20 et 60°C et avec des temps de réaction entre 8 et 24 heures.

28. Procédé selon la revendication 1, **caractérisé en ce qu'**un mélange du complexe de gadolinium de l'acide 10-[4-carboxy-1-méthyl-2-oxo-3-azabutyl]-1,4,7,10-tétraazacyclododécane-1,4,7-triacétique et de 1 à 2 équivalent (s) de chlorure de lithium est préparé et isolé et que celui-ci est ensuite traité au préalable par l'addition de 4-nitrophénol et de dicyclohexylcarbodiimide dans du DMSO à température ambiante et avec des temps de réaction entre 3 et 12 heures, et est ensuite mis à réagir éventuellement avec addition d'eau avec la polyamine 24-mère à base de triamide d'acide N,N,N',N',N",N"-hexakis[2-(trilysyl-amino)-éthyl]-trimésique entre 20 et 60°C et avec des temps de réaction entre 8 et 24 heures.
